# EUROPEAN PATENT SPECIFICATION

(11) **EP 3 345 951 B1**
(45) Date of publication and mention of the grant of the patent: **01.07.2020**
(21) Application number: 16841411.8
(22) Date of filing: 05.08.2016
(51) Int. Cl.: C08G 59/68, C09K 3/00, G03F 7/004, G03F 7/038

(54) **CURABLE COMPOSITION AND CURED ARTICLE USING SAME**
HÄRTBARE ZUSAMMENSETZUNG UND GEHÄRTETER ARTIKEL DAMIT
COMPOSITION DURCISSABLE ET ARTICLE DURCI L'UTILISANT

(30) Priority: 03.09.2015 JP 2015173487
(43) Date of publication of application: 11.07.2018
(73) Proprietor: San-Apro Limited, Kyoto-shi, Kyoto 605-0995 (JP)
(72) Inventor: FUKUNAGA, Noriya, Kyoto-shi, Kyoto 615-8245 (JP); TAKASHIMA, Yusaku, Kyoto-shi, Kyoto 615-8245 (JP); IKEDA, Takuya, Kyoto-shi, Kyoto 615-8245 (JP); SEIKE, Hideo, Kyoto-shi, Kyoto 615-8245 (JP)
(74) Representative: Maiwald Patent- und Rechtsanwaltsgesellschaft mbH
(86) International application number: PCT/JP2016/073064
(87) International publication number: WO 2017/038379

(56) References cited:
- EP-A1- 1 443 042
- EP-A1- 1 481 973
- EP-A1- 1 676 835
- WO-A1-01/12720
- WO-A2-02/08309
- JP-A- 2005 120 190
- JP-A- 2005 187 799
- JP-A- 2011 195 499
- JP-A- 2011 523 670
- JP-A- 2013 014 545
- JP-A- 2013 043 864
- REN K ET AL: "Tetrakis(pentafluorophenyl)gallates (I)", TETRAHEDRON LET, ELSEVIER, AMSTERDAM, NL, vol. 41, no. 45, 4 November 2000 (2000-11-04), pages 8669-8672, XP004236055, ISSN: 0040-4039, DOI: 10.1016/S0040-4039(00)01428-3
- KANGTAI REN ET AL.: 'Studies of Weekly Coordinating Anions Paired with Iodonium Cations' MACROMOLECULES vol. 35, no. 5, 30 January 2002, pages 1632 - 1637, XP002259287

## Description

### TECHNICAL FIELD

First, the present invention relates to a heat- or energy ray-curable composition for members that are required to have optical properties, the composition containing an onium gallate salt having a specific structure; and a cured article obtained by curing the same.

Second, the present invention relates to a chemically amplified negative photoresist composition for members that are required to have optical properties, the composition containing an onium gallate salt having a specific structure; and a cured article obtained by curing the same.

### BACKGROUND ART

Onium salts such as iodonium and sulfonium salts have been heretofore known as cationic polymerization initiators for curing a cationically polymerizable compound such as an epoxy compound by application of heat, light or an active energy ray such as an electron beam (see Patent Documents 1 to 10).

In addition, since such an onium salt generates an acid by application of heat or an active energy ray, the onium salt is also referred to as an acid generator, and also used for resists and photosensitive materials (Patent Documents 11 to 13).

Incidentally, although cationic polymerization initiators (acid generators) described in these specifications contain BF₄⁻, PF₆⁻, AsF₆⁻, or SbF₆⁻ as an anion, initiation performance for cationic polymerization varies with the kind of an anion and the respective initiators are improved in an ascending order of BF₄⁻ < PF₆⁻ < AsF₆⁻ < SbF₆⁻. However, applications of cationic polymerization initiators containing AsF₆⁻ or SbF₆⁻ being satisfactory in the polymerization-initiating ability are limited from a toxicity problem of As or Sb, and only an SbF₆⁻ salt is used in a limited field such as photofabrication. On that account, although a PF₆⁻ salt being poor in the polymerization-initiating ability is generally utilized, for example, in order to attain a curing rate almost comparable to that of an SbF₆⁻ salt, since the PF₆⁻ salt in an amount being nearly ten times the amount of the SbF₆⁻ salt needs to be added and the remaining amount of an unreacted initiator (acid generator), a solvent used as necessary for dissolving the initiator (acid generator) , or a decomposition product of the initiator is increased, there are problems that physical properties of a cured material are impaired, and moreover, a base material, facilities, and the like are liable to be corroded because of an increased amount of HF produced as a by-product by decomposition of the initiator. As such, a cationic polymerization initiator that is free from a toxic metal and has a cationic polymerization-initiating ability comparable to that of an SbF₆⁻ salt has been strongly required.

In members that are required to have optical properties, such as displays, optical waveguides and optical lenses, importance is placed on transparency of cured products cured by application of heat, light or irradiation with an active energy ray such as an electron beam, and transparency of cured products after a heat resistance test and after a humidity resistance test. In addition, applications requiring corrosion resistance to a remaining strong acid include members such as paint compositions, coating agents, ink compositions, inkjet ink compositions, resist films, liquid resists, negative resists, MEMS resists, negative photosensitive materials, various adhesives, molding materials, casting materials, putty materials, glass fiber impregnating agents, fillers, sealing agents, sealants, optical semiconductor (LED) sealants, optical waveguide materials, nano-imprint materials, stereolithography materials, micro-stereolithography materials, and ACF (anisotropic conductive films).

The present inventors have proposed a fluorinated alkylphosphonic acid onium salt-based acid generator (Patent Document 14) as a cationic polymerization initiator (acid generator) which does not contain a toxic metal, and has cationic polymerization performance and crosslinking reaction performance comparable to those of a SbF₆⁻ salt. However, a cured product including the acid generator has the problem that transparency is deteriorated particularly after a heat resistance test, and application of the acid generator to the above-mentioned members that are required to have optical properties has not progressed.

In addition, an onium salt having tetrakis(pentafluorophenyl)borate as an anion (Patent Document 15) is known as a cationic polymerization initiator (acid generator) which does not contain a toxic metal, and has cationic polymerization performance and crosslinking reaction performance comparable to those of a SbF₆⁻ salt. However, a cured product including the acid generator has the problem that transparency is deteriorated because HB(C₆F₅)₄ as a strong acid remaining particularly after a heat resistance test causes corrosion and coloring of a resin etc., and application of the acid generator to the above-mentioned members that are required to have optical properties has not progressed.

### PRIOR ART DOCUMENT

### PATENT DOCUMENTS

Patent Document 1: Japanese Patent Application Laid-Open (JP-A) No.50-151997
Patent Document 2: JP-A No.50-158680
Patent Document 3: JP-A No.02-178303
Patent Document 4: JP-A No.02-178303
Patent Document 5: US Patent (USP) No.4069054
Patent Document 6: USP No.4450360
Patent Document 7: USP No.4576999
Patent Document 8: USP No.4640967
Patent Document 9: Canada Patent No.1274646
Patent Document 10: European Patent Application Publication No.203829
Patent Document 11: JP-A No.2002-193925
Patent Document 12: JP-A No.2001-354669
Patent Document 13: JP-A No.2001-294570
Patent Document 14: WO2005-116038
Patent Document 15: JP-A No.2000-66385

WO 02/08309 and WO 01/12720 disclose a curable composition comprising an onium gallate salt.

### SUMMARY OF THE INVENTION

### PROBLEMS TO BE SOLVED BY THE INVENTION

In view of the above-mentioned background, a first object of the present invention is to provide a heat- or energy ray-curable composition including a cationic polymerization initiator (acid generator) which does not contain a toxic metal, has cationic polymerization performance and crosslinking reaction performance equivalent to or higher than those of tetrakis(F pentafluorophenyl)borate salt, and is capable of producing a cured product free from corrosion of members and free from the problem that transparency is deteriorated by corrosion of a resin because a strong acid does not remain particularly after a heat resistance test; and a cured article.

A second object of the present invention is to provide a chemically amplified negative photoresist composition including the above-mentioned acid generator; and a cured article.

### SOLUTIONS TO THE PROBLEMS

The present inventors have found an acid generator suitable for the above-mentioned objects.

That is, the present invention provides a heat- or energy ray-curable composition comprising a cationically polymerizable compound, and an acid generator containing an onium gallate salt represented by the following general formula (1); and a cured article obtained by curing the composition.

In addition, the present invention provides a chemically amplified negative photoresist composition comprising a component (A) including an acid generator, a component (B) as an alkali-soluble resin having a phenolic hydroxyl group, and a crosslinking agent component (C), the acid generator containing an onium gallate salt represented by the following general formula (1); and a cured article obtained by curing the photoresist composition. 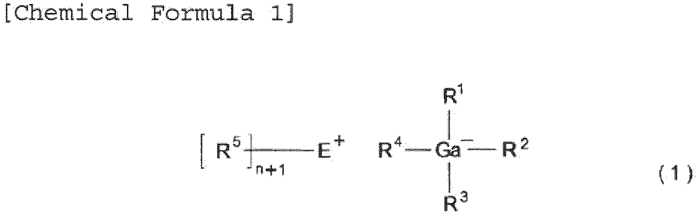
[In formula (1), R¹ to R⁴ each independently represent an alkyl group having 1 to 18 carbon atoms or Ar, with the proviso that at least one of R¹ to R⁴ is Ar;
Ar represents an aryl group having 6 to 14 carbon atoms (excluding the number of carbon atoms of the following substituents), and some of hydrogen atoms in the aryl group may be substituted with an alkyl group having 1 to 18 carbon atoms, a halogen atom-substituted alkyl group having 1 to 8 carbon atoms, an alkenyl group having 2 to 18 carbon atoms, an alkynyl group having 2 to 18 carbon atoms, an aryl group having 6 to 14 carbon atoms, a nitro group, a hydroxyl group, a cyano group, an alkoxy group or aryloxy group represented by -OR⁶, an acyl group represented by R⁷CO-, an acyloxy group represented by R⁸COO-, an alkylthio group or arylthio group represented by -SR⁹, an amino group represented by -NR¹⁰R¹¹, or a halogen atom;
R⁶ to R⁹ each represent an alkyl group having 1 to 8 carbon atoms, or an aryl group having 6 to 14 carbon atoms;
R¹⁰ and R¹¹ each represent a hydrogen atom, an alkyl group having 1 to 8 carbon atoms, or an aryl group having 6 to 14 carbon atoms;
E represents an n-valent element of Group 15 to Group 17 (IUPAC nomenclature);
n represents an integer of 1 to 3; and
R⁵ is an organic group bonded to E, the number of R⁵s is n + 1, (n + 1) R⁵s may be the same or different, and two or more R⁵s may be linked together directly or through -O-, -S-, -SO-, -SO₂-, -NH-, -CO-, -COO-, -CONH-, an alkylene group or a phenylene group to form a ring structure including the element E.]

The heat- or energy ray-curable composition of the present invention does not contain a highly toxic element such as Sb, and has cationic polymerization performance and crosslinking reaction performance comparable to those in the case of using a SbF₆⁻ salt, and a cured product cured by application of heat or an active energy ray such as an ultraviolet ray has favorable optical properties (transparency).

The chemically amplified negative photoresist composition of the present invention does not contain a highly toxic element such as Sb, and is excellent in UV curing performance, and a cured product cured by application of heat or an active energy ray such as an ultraviolet ray has favorable optical properties (transparency).

### MODE FOR CARRYING OUT THE INVENTION

Hereinafter, embodiments of the present invention will be described in detail.

The onium gallate salt-based acid generator according to the present invention is represented by the following general formula (1). 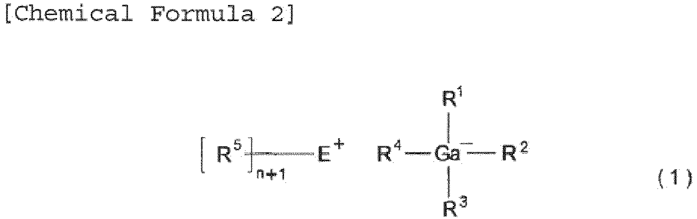
[In formula (1), R¹ to R⁴ each independently represent an alkyl group having 1 to 18 carbon atoms or Ar, with the proviso that at least one of R¹ to R⁴ is Ar;
Ar represents an aryl group having 6 to 14 carbon atoms (excluding the number of carbon atoms of the following substituents), and some of hydrogen atoms in the aryl group may be substituted with an alkyl group having 1 to 18 carbon atoms, a halogen atom-substituted alkyl group having 1 to 8 carbon atoms, an alkenyl group having 2 to 18 carbon atoms, an alkynyl group having 2 to 18 carbon atoms, an aryl group having 6 to 14 carbon atoms, a nitro group, a hydroxyl group, a cyano group, an alkoxy group or aryloxy group represented by -OR⁶, an acyl group represented by R⁷CO-, an acyloxy group represented by R⁸COO-, an alkylthio group or arylthio group represented by -SR⁹, an amino group represented by -NR¹⁰R¹¹, or a halogen atom;
R⁶ to R⁹ each represent an alkyl group having 1 to 8 carbon atoms, or an aryl group having 6 to 14 carbon atoms;
R¹⁰ and R¹¹ each represent a hydrogen atom, an alkyl group having 1 to 8 carbon atoms, or an aryl group having 6 to 14 carbon atoms;
E represents an n-valent element of Group 15 to Group 17 (IUPAC nomenclature);
n represents an integer of 1 to 3; and
R⁵ is an organic group bonded to E, the number of R⁵s is n + 1, (n + 1) R⁵s may be the same or different, and two or more R⁵s may be linked together directly or through -O-, -S-, -SO-, -SO₂-, -NH-, -CO-, -COO-, -CONH-, an alkylene group or a phenylene group to form a ring structure including the element E.]

Examples of the alkyl group having 1 to 18 carbon atoms for R¹ to R⁴ in general formula (1) include straight chain alkyl groups (such as methyl, ethyl, n-propyl, n-butyl, n-pentyl, n-octyl, n-decyl, n-dodecyl, n-tetradecyl, n-hexadecyl and n-octadecyl), branched chain alkyl groups (such as isopropyl, isobutyl, sec-butyl, tert-butyl, isopentyl, neopentyl, tert-pentyl, isohexyl, 2-ethylhexyl and 1,1,3,3-tetramethylbutyl), cycloalkyl groups (such as cyclopropyl, cyclobutyl, cyclopentyl and cyclohexyl), and crosslinked cyclic alkyl groups (such as norbornyl, adamantyl and pinanyl).

From the viewpoint of catalytic activity in a cationic polymerization reaction, those substituted with a halogen atom, a nitro group or a cyano group are preferable, and among them, those substituted with a fluorine atom are more preferable.

Examples of the aryl group having 6 to 14 carbon atoms (excluding the number of carbon atoms of the following substituents) for R¹ to R⁴ in general formula (1) include monocyclic aryl groups (such as a phenyl), fused polycyclic aryl groups (such as naphthyl, anthracenyl, phenanthrenyl, anthraquinolyl, fluorenyl and naphthoquinolyl), and aromatic heterocyclic hydrocarbon groups (such as monocyclic heterocyclic rings such as thienyl, furanyl, pyranyl, pyrrolyl, oxazolyl, thiazolyl, pyridyl, pyrimidyl and pyrazinyl; and fused polycyclic heterocyclic rings such as indolyl, benzofuranyl, isobenzofuranyl, benzothienyl, isobenzothienyl, quinolyl, isoquinolyl, quinoxalinyl, quinazolinyl, carbazolyl, acridinyl, phenothiazinyl, phenazinyl, xanthenyl, thianthrenyl, phenoxazinyl, phenoxathiinyl, chromanyl, isochromanyl, coumarinyl, dibenzothienyl, xanthonyl, thioxanthonyl and dibenzofuranyl).

The aryl group is not limited to those described above, and some of hydrogen atoms in the aryl group may be substituted with an alkyl group having 1 to 18 carbon atoms, a halogen atom-substituted alkyl group having 1 to 8 carbon atoms, an alkenyl group having 2 to 18 carbon atoms, an alkynyl group having 2 to 18 carbon atoms, an aryl group having 6 to 14 carbon atoms, a nitro group, a hydroxyl group, a cyano group, an alkoxy group or aryloxy group represented by -OR⁶, an acyl group represented by R⁷CO-, an acyloxy group represented by R⁸COO-, an alkylthio group or arylthio group represented by -SR⁹, an amino group represented by -NR¹⁰R¹¹, or a halogen atom.

Examples of the alkenyl group having 2 to 18 carbon atoms in the above-mentioned substituents include straight chain or branched chain alkenyl groups (such as vinyl, allyl, 1-propenyl, 2-propenyl, 1-butenyl, 2-butenyl, 3-butenyl, 1-methyl-1-propenyl, 1-methyl-2-propenyl, 2-methyl-1-propenyl and 2-methyl-2-propenyl), cycloalkenyl groups (2-cyclohexenyl and 3-cyclohexenyl) and aryl alkenyl groups (such as styryl and cinnamyl).

Examples of the alkynyl group having 2 to 18 carbon atoms in the above-mentioned substituents include straight chain or branched chain alkynyl groups (such as ethynyl, 1-propynyl, 2-propynyl, 1-butynyl, 2-butynyl, 3-butynyl, 1-methyl-2-propynyl, 1,1-dimethyl-2-propynyl, 1-pentynyl, 2-pentynyl, 3-pentynyl, 4-pentynyl, 1-methyl-2-butynyl, 3-methyl-1-butynyl, 1-decynyl, 2-decynyl, 8-decynyl, 1-dodecynyl, 2-dodecynyl and 10-dodecynyl) and arylalkynyl groups (such as phenylethynyl).

Examples of the halogen atom-substituted alkyl group having 1 to 8 carbon atoms in the above-mentioned substituents include straight chain alkyl groups (such as trifluoromethyl, trichloromethyl, pentafluoroethyl, 2,2,2-trichloroethyl, 2,2,2-trifluoroethyl, 1,1-difluoroethyl, heptafluoro-n-propyl, 1,1-difluoro-n-propyl, 3,3,3-trifluoro-n-propyl, nonafluoro-n-butyl, 3,3,4,4,4-pentafluoro-n-butyl, perfluoro-n-pentyl and perfluoro-n-octyl), branched alkyl groups (such as hexafluoroisopropyl, hexachloroisopropyl, hexafluoroisobutyl and nonafluoro-tert-butyl), cycloalkyl groups (such as pentafluorocyclopropyl, nonafluorocyclobutyl, perfluorocyclopentyl and perfluorocyclohexyl) and crosslinked cyclic alkyl groups (such as perfluoroadamantyl).

Examples of R⁶ to R¹¹ of the alkoxy group represented by -OR⁶, the acyl group represented by R⁷CO-, the acyloxy group represented by R⁸COO-, the alkylthio group represented by -SR⁹ and the amino group represented by - NR¹⁰R¹¹ in the above-mentioned substituents include alkyl groups having 1 to 8 carbon atoms, and specific examples thereof include alkyl groups having 1 to 8 carbon atoms among the above-mentioned alkyl groups.

Examples of R⁶ to R¹¹ of the aryloxy group represented by -OR⁶, the acyl group represented by R⁷CO-, the acyloxy group represented by R⁸COO-, the arylthio group represented by -SR⁹ and the amino group represented by -NR¹⁰R¹¹ in the above-mentioned substituents include aryl groups having 6 to 14 carbon atoms, and specific examples thereof include the above-mentioned aryl groups having 6 to 14 carbon atoms.

Examples of the alkoxy group represented by -OR⁶ include methoxy, ethoxy, n-propoxy, iso-propoxy, n-butoxy, sec-butoxy, tert-butoxy, n-pentoxy, iso-pentoxy, neo-pentoxy and 2-methylbutoxy.

Examples of the aryloxy group represented by -OR⁶ include phenoxy and naphthoxy.

Examples of the acyl group represented by R⁷CO-include acetyl, propanoyl, butanoyl, pivaloyl and benzoyl.

Examples of the acyloxy group represented by R⁸COO-include acetoxy, butanoyloxy and benzoyloxy.

Examples of the alkylthio group represented by -SR⁹ include methylthio, ethylthio, butylthio, hexylthio and cyclohexylthio.

Examples of the arylthio group represented by -SR⁹ include phenylthio and naphthylthio.

Examples of the amino group represented by -NR¹⁰R¹¹ include methylamino, ethylamino, propylamino, dimethylamino, diethylamino, methylethylamino, dipropylamino, and piperidino.

Examples of the halogen atom include a fluorine atom, a chlorine atom, a bromine atom and an iodine atom.

In these substituents, from the viewpoint of catalytic activity in a cationic polymerization reaction, halogen atom-substituted alkyl groups having 1 to 8 carbon atoms, halogen atoms, nitro groups and cyano groups are preferable, and halogen atom-substituted alkyl groups having 1 to 8 carbon atoms and a fluorine atom are more preferable.

R⁵s in formula (1) each represent an organic group bonded to E, and may be the same or different. Examples of R⁵ include aryl groups having 6 to 14 carbon atoms, alkyl groups having 1 to 18 carbon atoms, alkenyl groups having 2 to 18 carbon atoms and alkynyl groups having 2 to 18 carbon atoms, and the aryl group may be further substituted with an alkyl group having 1 to 18 carbon atoms, an alkenyl group having 2 to 18 carbon atoms, an alkynyl group having 2 to 18 carbon atoms, an aryl group having 6 to 14 carbon atoms, a nitro group, a hydroxyl group, a cyano group, an alkoxy group or aryloxy group represented by -OR⁶, an acyl group represented by R⁷CO-, an acyloxy group represented by R⁸COO-, an alkylthio group or arylthio group represented by -SR⁹, an amino group represented by -NR¹⁰R¹¹, or a halogen atom.

Examples of the aryl group having 6 to 14 carbon atoms, the alkyl group having 1 to 18 carbon atoms, the alkenyl group having 2 to 18 carbon atoms and the alkynyl group having 2 to 18 carbon atoms in the above-mentioned organic group include those described for R¹ to R⁴ in general formula (1).

In addition, two or more R⁵s may be linked together directly or through -O-, -S-, -SO-, -SO₂-, -NH-, -CO-,-COO-, -CONH-, an alkylene group or a phenylene group to form a ring structure containing the element A.

E in formula (1) represents an n-valent element of Group 15 to Group 17 (IUPAC nomenclature), and the element is bonded to the organic group R⁵ to form an onium ion [E⁺]. Among elements of Group 15 to Group 17, O (oxygen), N (nitrogen), P (phosphorus), S (sulfur) and I (iodine) are preferable, and corresponding onium ions include oxonium, ammonium, phosphonium, sulfonium and iodonium. Among them, ammonium, phosphonium, sulfonium and iodonium, which are stable and easy to handle, are preferable, and sulfonium and iodonium, which are excellent in cationic polymerization performance and crosslinking reaction performance, are more preferable.

n represents a valency of the element E, and is an integer of 1 to 3.

Specific examples of the oxonium ion include oxoniums such as trimethyloxonium, diethylmethyloxonium, triethyloxonium and tetramethylenemethyloxonium; pyryliniums such as 4-methylpyrylinium, 2,4,6-trimethylpyrylinium, 2,6-di-tert-butylpyrylinium and 2,6-diphenylpyrylinium; and chromeniums and isochromeniums such as 2,4-dimethylchromenium and 1,3-dimethylisochromenium.

Specific examples of the ammonium ion include tetraalkylammoniums such as tetramethylammonium, ethyltrimethylammonium, diethyldimethylammonium, triethylmethylammonium and tetraethylammonium; pyrrolidiniums such as N,N-dimethylpyrrolidinium, N-ethyl-N-methylpyrrolidinium and N,N-diethylpyrrolidinium; imidazoliniums such as N,N'-dimethylimidazolinium, N,N'-diethylimidazolinium, N-ethyl-N'-methylimidazolinium, 1,3,4-trimethylimidazolinium and 1,2,3,4-tetramethylimidazolinium; tetrahydropyrimidiniums such as N,N'-dimethyltetrahydropyrimidinium; morpholiniums such as N,N'-dimethylmorpholinium; piperidiniums such as N,N'-diethylpiperidinium; pyridiniums such as N-methylpyridinium, N-benzylpyridinium and N-phenacylpyridinium; imidazoliums such as N,N'-dimethylimidazolium; quinoliums such as N-methylquinolium, N-benzylquinolium and N-phenacylquinolium; isoquinoliums such as N-methylisoquinolium; thiazoniums such as benzylbenzothiazonium and phenacylbenzothiazonium; and acridiums such as benzylacridium and phenacylacridium.

Specific examples of the phosphonium ion include tetraarylphosphoniums such as tetraphenylphosphonium, tetra-p-tolylphosphonium, tetrakis(2-methoxyphenyl)phosphonium, tetrakis(3-methoxyphenyl)phosphonium and tetrakis(4-methoxyphenyl)phosphonium; triarylphosphoniums such as triphenylbenzylphosphonium, triphenylphenacylphosphonium, triphenylmethylphosphonium and triphenylbutylphosphonium; and tetraalkylphosphoniums such as triethylbenzylphosphonium, tributylbenzylphosphonium, tetraethylphosphonium, tetrabutylphosphonium, tetrahexylphosphonium, triethylphenacylphosphonium and tributylphenacylphosphonium.

Specific examples of the sulfonium ion include triarylsulfoniums such as triphenylsulfonium, tri-p-tolylsulfonium, tri-o-tolylsulfonium, tris(4-methoxyphenyl)sulfonium, 1-naphthyldiphenylsulfonium, 2-naphthyldiphenylsulfonium, tris(4-fluorophenyl)sulfonium, tri-1-naphthylsulfonium, tri-2-naphthylsulfonium, tris(4-hydroxyphenyl)sulfonium, 4-(phenylthio)phenyldiphenylsulfonium, 4-(p-tolylthio)phenyldi-p-tolylsulfonium, 4-(4-methoxyphenylthio)phenylbis(4-methoxyphenyl)sulfonium, 4-(phenylthio)phenylbis(4-fluorophenyl)sulfonium, 4-(phenylthio)phenylbis(4-methoxyphenyl)sulfonium, 4-(phenylthio)phenyldi-p-tolylsulfonium, [4-(4-biphenylylthio)phenyl]-4-biphenylylphenylsulfonium, [4-(2-thioxanthonylthio)phenyl]diphenylsulfonium, bis[4-(diphenylsulfonio)phenyl]sulfide, bis[4-{bis[4-(2-hydroxyethoxy)phenyl]sulfonio}phenyl]sulfide, bis{4-[bis(4-fluorophenyl)sulfonio]phenyl}sulfide, bis{4-[bis(4-methylphenyl)sulfonio]phenyl}sulfide, bis{4-[bis(4-methoxyphenyl)sulfonio]phenyl}sulfide, 4-(4-benzoyl-2-chlorophenylthio)phenylbis(4-fluorophenyl)sulfonium, 4-(4-benzoyl-2-chlorophenylthio)phenyldiphenylsulfonium, 4-(4-benzoylphenylthio)phenylbis(4-fluorophenyl)sulfonium, 4-(4-benzoylphenylthio)phenyldiphenylsulfonium, 7-isopropyl-9-oxo-10-thia-9,10-dihydroanthracene-2-yldi-p-tolylsulfonium, 7-isopropyl-9-oxo-10-thia-9,10-dihydroanthracene-2-yldiphenylsulfonium, 2-[(di-p-tolyl)sulfonio]thioxanthone, 2-[(diphenyl)sulfonio]thioxanthone, 4-(9-oxo-9H-thioxanthene-2-yl)thiophenyl-9-oxo-9H-thioxanthene-2-ylphenylsulfonium, 4-[4-(4-tert-butylbenzoyl)phenylthio]phenyldi-p-tolylsulfonium, 4- [4-(4-tert-butylbenzoyl)phenylthio]phenyldiphenylsulfonium, 4-[4-(benzoylphenylthio)]phenyldi-p-tolylsulfonium, 4-[4-(benzoylphenylthio)]phenyldiphenylsulfonium, 5-(4-methoxyphenyl)thiaanthrenium, 5-phenylthiaanthrenium, 5-tolylthiaanthrenium, 5-(4-ethoxyphenyl)thiaanthrenium and 5-(2,4,6-trimethylphenyl)thiaanthrenium; diarylsulfoniums such as diphenylphenacylsulfonium, diphenyl 4-nitrophenacylsulfonium, diphenylbenzylsulfonium and diphenylmethylsulfonium; monoarylsulfoniums such as phenylmethylbenzylsulfonium, 4-hydroxyphenylmethylbenzylsulfonium, 4-methoxyphenylmethylbenzylsulfonium, 4-acetocarbonyloxyphenylmethylbenzylsulfonium, 4-hydroxyphenyl(2-naphthylmethyl)methylsulfonium, 2-naphthylmethylbenzylsulfonium, 2-naphthylmethyl(1-ethoxycarbonyl)ethylsulfonium, phenylmethylphenacylsulfonium, 4-hydroxyphenylmethylphenacylsulfonium, 4-methoxyphenylmethylphenacylsulfonium, 4-acetocarbonyloxyphenylmethylphenacylsulfonium, 2-naphthylmethylphenacylsulfonium, 2-naphthyloctadecylphenacylsulfonium and 9-anthracenylmethylphenacylsulfonium; and trialkylsulfoniums such as dimethylphenacylsulfonium, phenacyltetrahydrothiophenium, dimethylbenzylsulfonium, benzyltetrahydrothiophenium and octadecylmethylphenacylsulfonium.

Specific examples of the iodonium ion include iodonium ions such as diphenyliodonium, di-p-tolyliodonium, bis(4-dodecylphenyl)iodonium, bis(4-methoxyphenyl)iodonium, (4-octyloxyphenyl)phenyliodonium, bis(4-decyloxy)phenyliodonium, 4-(2-hydroxytetradecyloxy)phenylphenyliodonium, 4-isopropylphenyl(p-tolyl)iodonium and 4-isobutylphenyl(p-tolyl)iodonium.

Preferred examples of the anion structure of an acid generator represented by general formula (1) may include those represented by the following chemical formulae (A-1) to (A-5).

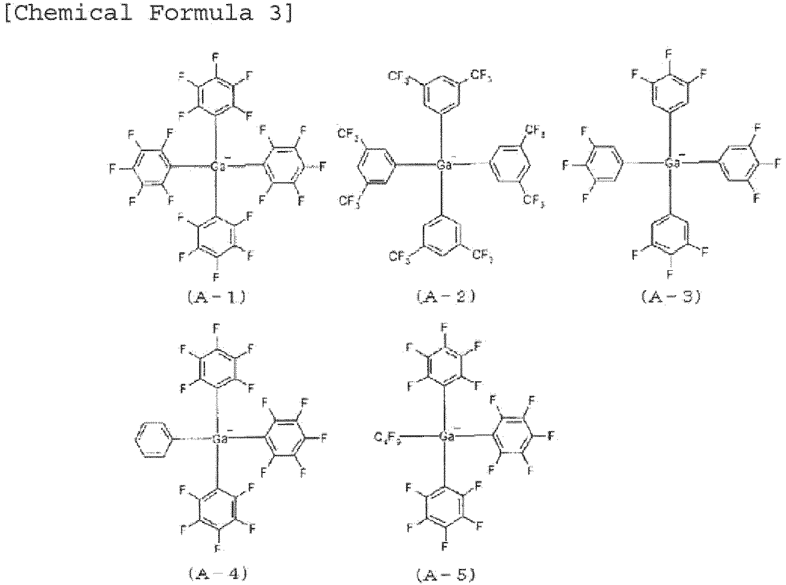

The amount of impurities contained in the curable composition is 4 parts by weight or less, preferably 0.01 to 2 parts by weight based on 100 parts by weight of the onium gallate salt. Decomposition of the gallate anion is promoted by impurities, and therefore when the amount of impurities is more than 4 parts by weight, curability is reduced, leading to deterioration of the physical properties of the cured article.

While the impurities are not limited, mention is made of a hydroxytris(pentafluorophenyl)gallate salt as one example of the impurities. The gallate anion salt in the present invention can be synthesized by a known method. For example, the gallate anion salt can be obtained by reacting gallium (III) chloride with an organometallic compound such as an organolithium compound or an organomagnesium compound (Tetrahedron 58 (2002) 5267-5273). This gallate anion salt inevitably contains a hydroxytris(pentafluorophenyl)gallate salt as an impurity because it is deliquescent, and thus reacts with water in air to generate a hydroxytris(pentafluorophenyl)gallate salt as a byproduct. Therefore, a curable composition containing the onium gallate salt obtained by salt exchanging an onium salt and the gallate anion salt containing a hydroxytris(pentafluorophenyl)gallate salt and represented by formula (1) in the present invention also contains hydroxytris(pentafluorophenyl)gallate.

¹H-NMR, ¹³C-NMR, ¹⁹F-NMR and high performance liquid chromatography (HPLC) are used as a method for analysis of the amount of impurities in the onium gallate salt as defined in the present invention. The measurement conditions for ¹H-NMR, ¹³C-NMR and ¹⁹F-NMR are as follows. Apparatus: AL-300 (manufactured by JEOL Ltd.), solvent: dimethylsulfoxide. The measurement conditions for HPLC are as follows. Apparatus: model (L-2130), manufacturer (Hitachi, Ltd.); column: (Ph-3), manufacturer (GL Sciences Inc.), moving bed: solution of methanol : water : sodium perchlorate-hydrate = 600 : 68 : 20, detector : UV (210 nm), injection amount 10 µl, column temperature 40°C.

For ensuring that the onium gallate salt (acid generator) represented by formula (1) is easily dissolved in a cationically polymerizable compound or chemically amplified negative resist composition, the onium gallate salt (acid generator) may be previously dissolved in a solvent which does not hinder polymerization or crosslinking reaction.

Examples of the solvent include carbonates such as propylene carbonate, ethylene carbonate, 1,2-butylene carbonate, dimethyl carbonate, and diethyl carbonate; ketones such as acetone, methyl ethyl ketone, cyclohexanone, methyl isoamyl ketone, and 2-heptanone; polyhydric alcohols and derivatives thereof, such as monomethyl ethers, monoethyl ethers, monopropyl ethers, monobutyl ethers, or monophenyl ethers of ethylene glycol, ethylene glycol monoacetate, diethylene glycol, diethylene glycol monoacetate, propylene glycol, propylene glycol monoacetate, dipropylene glycol, and dipropylene glycol monoacetate; cyclic ethers such as dioxane; esters such as ethyl formate, methyl lactate, ethyl lactate, methyl acetate, ethyl acetate, butyl acetate, methyl pyruvate, methyl acetoacetate, ethyl acetoacetate, ethyl pyruvate, ethyl ethoxyacetate, methyl methoxypropionate, ethyl ethoxypropionate, methyl 2-hydroxypropionate, ethyl 2-hydroxypropionate, ethyl 2-hydroxy-2-methylpropionate, methyl 2-hydroxy-3-methylbutanoate, 3-methoxybutyl acetate, and 3-methyl-3-methoxybutyl acetate; and aromatic hydrocarbons such as toluene and xylene.

When a solvent is used, the amount of the solvent used is preferably from 15 to 1000 parts by weight, more preferably from 30 to 500 parts by weight, based on 100 parts by weight of the onium gallate salt (acid generator) represented by formula (1) in the present invention. A single solvent may be used alone or two or more solvents may be used in combination.

The heat- or active energy ray-curable composition in the present invention (hereinafter, referred to as a curable composition) contains the above-mentioned acid generator and a cationically polymerizable compound.

Examples of the cationically polymerizable compound as a constituent component of the curable composition include cyclic ethers (such as epoxide and oxetane), ethylenically unsaturated compounds (such as vinyl ether and styrene), bicycloorthoesters, spiroorthocarbonates, and spiroorthoesters {(examples of the cationically polymerizable compound component in the active energy ray-curable composition include those described in JP-A No.11-060996, JP-A No.09-302269, JP-A No.2003-026993, JP-A No.2002-206017, JP-A No.11-349895, JP-A No.10-212343, JP-A No.2000-119306, JP-A No.10-67812, JP-A No.2000-186071, JP-A No.08-85775, JP-A No.08-134405, JP-A No.2008-20838, JP-A No.2008-20839, JP-A No.2008-20841, JP-A No.2008-26660, JP-A No.2008-26644, JP-A No.2007-277327, "Photopolymer Handbook" edited by The Technical Association of Photopolymers, Japan (1989, Kogyo Chosakai Publishing, Co., Ltd.), "UV/EB Koka Gijutsu" (Technology of UV/EB Curing), edited by Sogo Gijutsu Center (1982, Sogo Gijutsu Center), "UV/EB Koka Zairyo" (UV/EB Curable Materials), edited by RadTech Japan (1992, CMC), "UV-Koka niokeru Koka-Furyo/Sogai-Genin to Sonotaisaku" (Causes of UV Curing Defects/Inhibition and Countermeasures Therefor), edited by TECHNICAL INFORMATION INSTITUTE (2003, TECHNICAL INFORMATION INSTITUTE CO., LTD.), Journal of the Japan Society of Colour Material, 68, (5), 286-293 (1995), and Fine Chemical, 29, (19), 5-14 (2000); and each of these components may be used as a cationically polymerizable compound in a thermosetting composition}.

Known epoxides and the like may be used as epoxides, example of which include aromatic epoxides, alicyclic epoxides, and aliphatic epoxides.

Examples of the aromatic epoxides include glycidyl ethers of monohydric or polyhydric phenols having at least one aromatic ring (such as phenol, bisphenol A, phenol novolac, and alkylene oxide adducts thereof).

Examples of the alicyclic epoxides include compounds obtained by epoxidation of compounds having at least one cyclohexene or cyclopentene ring with an oxidizing agent (such as 3,4-epoxycyclohexylmethyl-3,4-epoxycyclohexanecarboxylate).

Examples of the aliphatic epoxides include polyglycidyl ethers of aliphatic polyhydric alcohols or alkylene oxide adducts thereof (such as 1,4-butanediol diglycidyl ether and 1,6-hexanediol diglycidyl ether), polyglycidyl esters of aliphatic polybasic acids (such as diglycidyl tetrahydrophthalate), and epoxidized long-chain unsaturated compounds (such as epoxidized soybean oil and epoxidized polybutadiene).

Known oxetanes and the like may be used as oxetanes, examples of which include 3-ethyl-3-hydroxymethyloxetane, 2-ethylhexyl(3-ethyl-3-oxetanylmethyl)ether, 2-hydroxyethyl(3-ethyl-3-oxetanylmethyl)ether, 2-hydroxypropyl(3-ethyl-3-oxetanylmethyl)ether, 1,4-bis[(3-ethyl-3-oxetanylmethoxy) methyl]benzene, oxetanylsilsesquioxetane, and phenol novolac oxetane.

Known cationically polymerizable monomers and the like may be used as ethylenically unsaturated compounds, examples of which include aliphatic monovinyl ethers, aromatic monovinyl ethers, polyfunctional vinyl ethers, styrenes, and cationically polymerizable nitrogen-containing monomers.

Examples of the aliphatic monovinyl ethers include methyl vinyl ether, ethyl vinyl ether, butyl vinyl ether, and cyclohexyl vinyl ether.

Examples of the aromatic monovinyl ethers include 2-phenoxyethyl vinyl ether, phenyl vinyl ether, and p-methoxyphenyl vinyl ether.

Examples of the polyfunctional vinyl ethers include butanediol-1,4-divinyl ether and triethylene glycol divinyl ether.

Examples of the styrenes include styrene, α-methylstyrene, p-methoxystyrene, and p-tert-butoxystyrene.

Examples of the cationically polymerizable nitrogen-containing monomers include N-vinylcarbazole and N-vinylpyrrolidone.

Examples of the bicycloorthoesters include 1-phenyl-4-ethyl-2,6,7-trioxabicyclo[2.2.2]octane and 1-ethyl-4-hydroxymethyl-2,6,7-trioxabicyclo[2.2.2]octane.

Examples of the spiroorthocarbonates include 1,5,7,11-tetraoxaspiro[5.5]undecane and 3,9-dibenzyl-1,5,7,11-tetraoxaspiro[5,5]undecane.

Examples of the spiroorthoesters include 1,4,6-trioxaspiro[4.4]nonane, 2-methyl-1,4,6-trioxaspiro[4.4]nonane, and 1,4,6-trioxaspiro[4.5]decane.

Further, a polyorganosiloxane having at least one cationically polymerizable group in one molecule can be used (those described in JP-A No.2001-348482, JP-A No.2000-281965, JP-A No.7-242828, JP-A No.2008-195931, Journal of Polym. Sci., Part A, Polym. Chem., Vol. 28, 497 (1990) and so on).

These polyorganosiloxanes may be any one of straight chain, branched chain and cyclic polyorganosiloxanes, or a mixture thereof.

Among these cationically polymerizable compounds, epoxides, oxetanes, and vinyl ethers are preferred, epoxides and oxetane are more preferred, and alicyclic epoxides and oxetanes are particularly preferred. These cationically polymerizable compounds may be used alone or in combination of two or more.

The content of the onium gallate salt represented by general formula (1) (acid generator) of the invention in an energy ray-curable composition is preferably from 0.05 to 20 parts by weight, more preferably from 0.1 to 10 parts by weight, based on 100 parts by weight of the cationically polymerizable compound. Within the range, the cationically polymerizable compound can be more sufficiently polymerized, so that the physical properties of the cured product can be further improved. It will be understood that the content may be determined taking into account various factors such as the properties of the cationically polymerizable compound, the type and irradiation dose of the active energy ray, the temperature, the curing time, the humidity, and the thickness of the coating film, and is not limited to the above range.

If necessary, the curable composition of the invention may contain known additives (such as a sensitizer, a pigment, a filler, an electroconductive particle, an antistatic agent, a flame retardant, an anti-foaming agent, a fluidity controlling agent, a light stabilizer, an antioxidant, a tackifier, an ion scavenger, an anti-coloring agent, a solvent, a nonreactive resin, and a radically-polymerizable compound).

Known sensitizers (such as those described in JP-A No.11-279212 and JP-A No.09-183960) and the like may be used as such a sensitizer, examples of which include benzoquinones {such as 1,4-benzoquinone, 1,2-benzoquinone}; naphthoquinones {such as 1,4-naphthoquinone, 1,2-naphthoquinone}; anthraquinones {such as 2-ethylanthraquinone, 2-methylanthraquinone}; anthracenes {such as anthracene, 9,10-dibutoxyanthracene, 9,10-dimethoxyanthracene, 9,10-diethoxyanthracene, 2-ethyl-9,10-dimethoxyanthracene, and 9,10-dipropoxyanthracene}; pyrene; 1,2-benzanthracene; perylene; tetracene; coronene; thioxanthones {such as thioxanthone, 2-methylthioxanthone, 2-ethylthioxanthone, 2-chlorothioxanthone, 2-isopropylthioxanthone, and 2,4-diethylthioxanthone}; phenothiazine {such as phenothiazine, N-methylphenothiazine, N-ethylphenothiazine, and N-phenylphenothiazine}; xanthone; naphthalenes {such as 1-naphthol, 2-naphthol, 1-methoxynaphthalene, 2-methoxynaphthalene, 1,4-dihydroxynaphthalene, and 4-methoxy-1-naphthol}; ketones {such as dimethoxyacetophenone, diethoxyacetophenone, 2-hydroxy-2-methyl-1-phenylpropan-1-one, 4'-isopropyl-2-hydroxy-2-methylpropiophenone, and 4-benzoyl-4'-methyldiphenylsulfide}; carbazoles {such as N-phenylcarbazole, N-ethylcarbazole, poly-N-vinylcarbazole, and N-glycidylcarbazole}; chrysenes {such as 1,4-dimethoxychrysene and 1,4-di-α-methylbenzyloxychrysene}; and phenanthrenes {such as 9-hydroxyphenanthrene, 9-methoxyphenanthrene, 9-hydroxy-10-methoxyphenanthrene, and 9-hydroxy-10-ethoxyphenanthrene}.

When a sensitizer is contained, the content of the sensitizer is preferably from 1 to 300 parts by weight, more preferably from 5 to 200 parts by weight, based on 100 parts of the acid generator.

Known pigments and the like may be used as pigments, examples of which include inorganic pigments (such as titanium oxide, iron oxide, and carbon black) and organic pigments (such as azo pigments, cyanine pigments, phthalocyanine pigments, and quinacridone pigments).

When a pigment is contained, the content of the pigment is preferably from 0.5 to 400,000 parts by weight, more preferably from 10 to 150,000 parts by weight, based on 100 parts of the acid generator.

Known fillers and the like may be used as fillers, examples of which include fused silica, crystalline silica, calcium carbonate, aluminum oxide, aluminum hydroxide, zirconium oxide, magnesium carbonate, mica, talc, calcium silicate, and lithium aluminum silicate.

When a filler is contained, the content of the filler is preferably from 50 to 600,000 parts by weight, more preferably 300 to 200,000 parts by weight, based on 100 parts of the acid generator.

Known electroconductive particles can be used as electroconductive particles, and metal particles of Ni, Ag, Au, Cu, Pd, Pb, Sn, Fe, Ni, Al and the like, and plated metal particles obtained by further plating the above-mentioned metal particles with a metal, plated resin particles obtained by plating resin particles with a metal, or particles of a substance having conductivity, such as carbon, can be used.

When electroconductive particles are contained, the content of the electroconductive particles is preferably from 50 to 30000 parts by weight, more preferably from 100 to 20000 parts by weight, based on 100 parts of the acid generator.

Known antistatic agents and the like may be used as antistatic agents, examples of which include nonionic antistatic agents, anionic antistatic agents, cationic antistatic agents, ampholytic antistatic agents, and high molecular weight antistatic agents.

When an antistatic agent is contained, the content of the antistatic agent is preferably from 0.1 to 20,000 parts by weight, more preferably from 0.6 to 5,000 parts by weight, based on 100 parts of the acid generator.

Known flame retardants and the like may be used as flame retardants, examples of which include inorganic flame retardants {such as antimony trioxide, antimony pentoxide, tin oxide, tin hydroxide, molybdenum oxide, zinc borate, barium metaborate, red phosphorus, aluminum hydroxide, magnesium hydroxide, and calcium aluminate}; bromine flame retardants {such as tetrabromophthalic anhydride, hexabromobenzene, and decabromobiphenyl ether}; and phosphate flame retardants {such as tris(tribromophenyl) phosphate}.

When a flame retardant is contained, the content of the flame retardant is preferably from 0.5 to 40,000 parts by weight, more preferably from 5 to 10,000 parts by weight, based on 100 parts of the acid generator.

Known anti-foaming agents and the like may be used as anti-foaming agents, examples of which include alcoholic anti-foaming agents, metallic soap anti-foaming agents, phosphate anti-foaming agents, fatty acid ester anti-foaming agents, polyether anti-foaming agents, silicone anti-foaming agents, and mineral oil anti-foaming agents.

Known fluidity controlling agents and the like may be used as fluidity controlling agents, examples of which include hydrogenated castor oil, oxidized polyethylene, organic bentonite, colloidal silica, amide wax, metallic soap, and acrylic ester polymers.

Known light stabilizers and the like may be used as light stabilizers, examples of which include ultraviolet absorbing stabilizers {such as benzotriazole, benzophenone, salicylates, cyanoacrylates, and derivatives thereof}; radical scavenging stabilizers {such as hindered amines}; and quenching stabilizers {such as nickel complexes}.

Known antioxidants and the like may be used as antioxidants, examples of which include phenolic antioxidants (such as monophenolic, bisphenolic, and macromolecular phenolic antioxidants), sulfur-based antioxidants, and phosphorus-based antioxidants. Known tackifiers and the like may be used as tackifiers, example of which include coupling agents, silane coupling agents, and titanium coupling agents.

Known ion scavenger and the like may be used as ion scavenger, examples of which include organoaluminum (such as alkoxyaluminum and phenoxyaluminum).

Known anti-coloring agents and the like may be used as anti-coloring agents, and antioxidants are generally effective, examples of which include phenolic antioxidants (such as monophenolic, bisphenolic, and macromolecular phenolic antioxidants), sulfur-based antioxidants, and phosphorus-based antioxidants.

When an anti-foaming agent, a fluidity controlling agent, a light stabilizer, an antioxidant, a tackifier, an ion scavenger, or an anti-coloring agent is contained, the content of each material is preferably from 0.1 to 20,000 parts by weight, more preferably from 0.5 to 5,000 parts by weight, based on 100 parts of the acid generator.

Any solvent that can be used to dissolve the cationically polymerizable compound or to control the viscosity of the energy ray-curable composition may be used as solvents, examples of which include those listed for the above acid generator.

When a solvent is contained, the content of the solvent is preferably from 50 to 2,000,000 parts by weight, more preferably from 200 to 500,000 parts by weight, based on 100 parts of the acid generator.

Examples of the nonreactive resin include polyester, polyvinyl acetate, polyvinyl chloride, polybutadiene, polycarbonate, polystyrene, polyvinyl ether, polyvinyl butyral, polybutene, hydrogenated styrene-butadiene block copolymers, copolymers of (meth)acrylic acid esters, and polyurethane. The number average molecular weight of these resins is preferably from 1,000 to 500,000, more preferably from 5,000 to 100,000 (the number average molecular weight is a value measured by a general method such as GPC).

When a nonreactive resin is contained, the content of the nonreactive resin is preferably from 5 to 400,000 parts by weight, more preferably from 50 to 150,000 parts by weight, based on 100 parts of the acid generator.

When a nonreactive resin is contained, it is preferably dissolved in advance in a solvent so that it can be easily dissolved in the cationically polymerizable compound or the like.

Known radically-polymerizable compounds and the like may be used as radically-polymerizable compounds {such as those described in "Photopolymer Handbook" edited by The Technical Association of Photopolymers, Japan (1989, Kogyo Chosakai Publishing, Co., Ltd.), "UV/EB Koka Gijutsu" (Technology of UV/EB Curing), edited by Sogo Gijutsu Center (1982, Sogo Gijutsu Center), "UV/EB Koka Zairyo" (UV/EB Curable Materials), edited by RadTech Japan (1992, CMC), and "UV-Koka niokeru Koka-Furyo/Sogai-Genin to Sonotaisaku" (Causes of UV Curing Defects/Inhibition and Remedies Therefor), edited by TECHNICAL INFORMATION INSTITUTE (2003, TECHNICAL INFORMATION INSTITUTE CO., LTD.)}, examples of which include monofunctional monomers, bifunctional monomers, polyfunctional monomers, epoxy (meth)acrylate, polyester (meth)acrylate, and urethane (meth)acrylate.

When a radically-polymerizable compound is contained, the content of the radically-polymerizable compound is preferably from 5 to 400,000 parts by weight, more preferably from 50 to 150,000 parts by weight, based on 100 parts of the acid generator.

When a radically-polymerizable compound is contained, a radical polymerization initiator initiating polymerization with heat or light is preferably used so that the compound can be polymerized by radical polymerization.

Known radical polymerization initiators and the like may be used as radical polymerization initiators, examples of which include thermal radical polymerization initiators (such as organic peroxides and azo compounds) and photoradical polymerization initiators (such as acetophenone-based initiators, benzophenone-based initiators, Michler's ketone-based initiators, benzoin-based initiators, thioxanthone-based initiators, and acylphosphine-based initiators.

When a radical polymerization initiator is contained, the content of the radical polymerization initiator is preferably from 0.01 to 20 parts by weight, more preferably from 0.1 to 10 parts by weight, based on 100 parts of the radically-polymerizable compound.

The curable composition of the invention may be prepared by uniformly mixing and dissolving the cationically polymerizable compound, the acid generator, and if necessary an optional additive(s) at room temperature (about 20 to 30°C) or if necessary, under heating (about 40 to 90°C), or by further kneading them with a triple-roll mill or the like.

Among the curable composition of the invention, the energy ray-curable composition of the invention may be cured by irradiation with energy rays so that a cured product can be obtained.

The energy ray may be of any energy ray as long as it has an energy to induce the decomposition of the sulfonium salt of the invention, preferred examples of which include energy rays in the ultraviolet to visible light region (wavelength: from about 100 to about 800 nm) obtained from a low pressure-, medium pressure-, high pressure-, or ultra high pressure-mercury lamp, a metal halide lamp, an LED lamp, a xenon lamp, a carbon arc lamp, a fluorescent lamp, a semiconductor solid-state laser, an argon laser, a He-Cd laser, a KrF excimer laser, an ArF excimer laser, or an F₂ laser. Radiations with a high energy, such as electron beams or X-rays may also be used as the energy rays.

While the energy ray irradiation time is influenced by the intensity of the energy rays or the permeability of the energy rays to the energy ray-curable composition, an energy ray exposure time of about 0.1 to 10 seconds is enough at room temperature (about 20 to 30°C) . However, if the permeability of the energy rays is low or if the thickness of the energy ray-curable composition is large, for example, it is sometimes preferred to spend more time. Most energy ray-curable compositions are cured by cationic polymerization in 0.1 seconds to several minutes after the irradiation with energy rays. If necessary, however, post-curing may be performed by heating at a temperature of room temperature (about 20 to 30°C) to 250°C for several seconds to several hours after the irradiation with energy rays.

Among the curable compositions of the present invention, a thermosetting composition can produce a cured article by generating an acid from an acid generator upon heating, and subjecting a cationically polymerizable compound to a polymerization or crosslinking reaction. The temperature necessary for curing is not particularly limited as long as curing sufficiently proceeds, and a base material is not deteriorated, but it is preferably 50°C to 300°C, more preferably 60°C to 250°C The heating time varies depending on the heating temperature, but it is preferably several minutes to several hours from the viewpoint of productivity.

Here, the base material is a material to be coated or filled with the curable composition of the present invention, and a known material can be appropriately used. Examples of the base material in the present invention include resin films such as PET films, polypropylene films and polyimide films, metal foils such as aluminum foils, substrates of glass, copper, aluminum and the like, devices, light emitting diode elements, transistors and integrated circuits, and also include elements or circuits formed on the substrates described above.

Specific applications of the curable composition of the present invention include paints, coating agents, various coating materials (hard coats, anti-fouling coating materials, anti-fogging coating materials, anti-corrosion coating materials, optical fibers and the like), back surface treatment agents for pressure sensitive adhesive tapes, release coating materials of release sheets for pressure sensitive adhesive labels (release papers, release plastic films, release metal foils and the like), printing plates, dental materials (dental formulations and dental composites), ink compositions, inkjet ink compositions, positive resists (for formation of connection terminals and wiring patterns in production of electronic components such as circuit boards, CSP and MEMS elements), resist films, liquid resists and negative resists (permanent film materials of surface protecting films, interlayer dielectric films, planarizing films for semiconductor elements and transparent electrodes for FPD (ITO, IZO and GZO), etc.), resists for MEMS, positive photosensitive materials, negative photosensitive materials, various adhesives (various temporary fixing agents for electronic components, adhesives for HDD, adhesives for pick-up lenses, functional films for FPD (polarizing plates, antireflection films and the like), insulating films for circuit formation and semiconductor sealing, anisotropic electroconductive adhesives (ACA), films (ACF), pastes (ACP) and the like), holographic resins, FPD materials (color filters, black matrices, partition wall materials, photospacers, ribs, orientation films for liquid crystals, sealing agents for FPD and the like), optical members, molding materials (for building materials, optical components and lenses), casting materials, putty materials, glass fiber impregnating agents, fillers, sealing materials, flip-chips, chip sealants for COF etc., sealants for packages such as CSP or BGA, photosemiconductor (LED) sealants, optical waveguide materials, nano-imprint materials, stereolithography materials, and micro-stereolithography materials. In particular, the resulting cured product is excellent in electrical properties (insulation reliability), and is therefore most suitable for electronic component/circuit forming material applications.

The acid generator of the invention, which can generate a strong acid upon irradiation with light, may also be used as a acid generator for known chemically amplified resist materials (such as those described in JP-A No.2003-267968, JP-A No.2003-261529, and JP-A No.2002-193925).

The chemically amplified negative photoresist composition of the invention comprises an ingredient (A) including the acid generator of the invention, which is a compound generating an acid when irradiated with light or a radiation; an alkali-soluble resin (B) having a phenolic hydroxyl group; and a crosslinking agent (C).

In the chemically amplified negative photoresist composition of the present invention, the ingredient (A) may be used in combination with a conventionally known additional acid generator. Examples of the additional acid generator include onium salt compounds, sulfone compounds, sulfonate compounds, sulfonimide compounds, disulfonyl diazomethane compounds, disulfonylmethane compounds, oxime sulfonate compounds, hydrazine sulfonate compounds, triazine compounds, and nitrobenzyl compounds, as well as organohalogen compounds and disulfone.

The conventionally known additional acid generator is preferably at least one selected from the group of an onium compound, a sulfonimide compound, a diazomethane compound, and an oxime sulfonate compound.

When such a conventionally known additional acid generator is used in combination, the amount of the additional acid generator used is generally from 10 to 900 parts by weight, preferably from 25 to 400 parts by weight, based on 100 parts by weight of the acid generator of the present invention, although the amount thereof used may be arbitrary.

The content of the above ingredient (A) is preferably from 0.01 to 10% by weight, in the solids of the chemically amplified negative photoresist composition.

### Alkali-Soluble Resin (B) Having Phenolic Hydroxyl Group

In the invention, examples of the "alkali-soluble resin having a phenolic hydroxyl group" (hereinafter referred to as "phenolic resin (B)"), which may be used, include a novolac resin, polyhydroxystyrene, a polyhydroxystyrene copolymer, a copolymer of hydroxystyrene and styrene, a copolymer of hydroxystyrene, styrene, and a (meth)acrylic acid derivative, a phenol-xylylene glycol condensate resin, a cresol-xylylene glycol condensate resin, a phenol-dicyclopentadiene condensate resin and a polyimide resin having a phenolic hydroxyl group. Among them, preferred are a novolac resin, polyhydroxystyrene, a polyhydroxystyrene copolymer, a copolymer of hydroxystyrene and styrene, a copolymer of hydroxystyrene, styrene, and a (meth)acrylic acid derivative, and a phenol-xylylene glycol condensate resin. These phenolic resins (B) may be used alone or in a mixture of two or more.

The phenolic resin (B) may also contain a low-molecular-weight phenolic compound as a partial component.

Examples of the above low-molecular-weight phenolic compound include 4,4'-dihydroxydiphenylmethane and 4,4'-dihydroxydiphenyl ether.

### Crosslinking Agent (C)

In the invention, the "crosslinking agent" (hereinafter also referred to as "crosslinking agent (C)") is not particularly limited as long as it acts as a crosslinking component (curing component) reactive with the aforesaid phenolic resin (B). Examples of the above crosslinking agent (C) include a compound having at least two alkyl-etherified amino groups in the molecule, a compound having at least two alkyl-etherified benzene skeletons in the molecule, an oxirane ring-containing compound, a thiirane ring-containing compound, an oxetanyl group-containing compound, and an isocyanate group-containing compound (including a blocked derivative).

Among these crosslinking agents (C), preferred are a compound having at least two alkyl-etherified amino groups in the molecule or an oxirane ring-containing compound. More preferably, a compound having at least two alkyl-etherified amino groups in the molecule is used in combination with an oxirane ring-containing compound.

In the invention, the amount of the crosslinking agent (C) formulated is preferably from 1 to 100 parts by weight, more preferably from 5 to 50 parts by weight, based on 100 parts by weight of the aforesaid phenolic resin (B). When the amount of the crosslinking agent (C) formulated is from 1 to 100 parts by weight, the curing reaction can sufficiently proceed, so that the resulting cured product can have a good pattern shape with a high resolution and have excellent heat resistance and electric insulation, which is preferred.

When a compound having alkyl-etherified amino groups is used in combination with an oxirane ring-containing compound, the content rate of the oxirane ring-containing compound is preferably 50% by weight or less, more preferably from 5 to 40% by weight, in particular, preferably from 5 to 30% by weight, based on the total amount of the compound having alkyl-etherified amino groups and the oxirane ring-containing compound, which is normalized as 100% by weight.

In this case, the resulting cured film also has excellent chemical resistance without losing the high resolution, which is preferred.

### Crosslinked Fine Particles (D)

The chemically amplified negative photoresist composition of the present invention may further contain crosslinked fine particles (D) for improving the durability and thermal shock resistance of the resulting cured product.

The crosslinked fine particles (D) are not particularly limited as long as the glass transition temperature (Tg) of a polymer that forms the crosslinked fine particles is 0°C or lower, but those obtained by copolymerizing a crosslinkable monomer having two or more unsaturated polymerizable groups (hereinafter, referred to simply as a "crosslinkable monomer") and one or more "additional monomers" selected in such a manner that the crosslinked fine particles (D) have a Tg of 0°C or lower are preferable.

In particular, it is preferable that the above-mentioned two or more additional monomers are used in combination, and at least one of the additional monomers has a functional group other than a polymerizable group, such as a carboxyl group, an epoxy group, an amino group, an isocyanate group or a hydroxyl group.

Examples of the crosslinkable monomer may include compounds having a plurality of polymerizable unsaturated groups, such as divinylbenzene, diallyl phthalate, ethylene glycol di(meth)acrylate, propylene glycol di(meth)acrylate, trimethylolpropane tri(meth)acrylate, pentaerythritol tri(meth)acrylate, polyethylene glycol di(meth)acrylate and polypropylene glycol di(meth)acrylate. Among them, divinylbenzene is preferable.

The amount of the above-mentioned crosslinkable monomer to be used in production of the above-mentioned crosslinked fine particles (D) is preferably from 1 to 20% by weight, more preferably from 1 to 10% by weight, especially preferably from 1 to 5% by weight, based on 100% by weight of all monomers to be used for copolymerization.

In addition, examples of the above-mentioned additional monomer may include diene compounds such as butadiene, isoprene, dimethylbutadiene, chloroprene and 1,3-pentadiene; unsaturated nitrile compounds such as (meth)acrylonitrile, α-chloroacrylonitrile, α-chloromethylacrylonitrile, α-methoxyacrylonitrile, α-ethoxyacrylonitrile, crotonic acid nitrile, cinnamic acid nitrile, itaconic acid dinitrile, maleic acid dinitrile and fumaric acid dinitrile; unsaturated amides such as (meth)acrylamide, dimethyl(meth)acrylamide, N,N'-methylenebis(meth)acrylamide, N,N'-ethylenebis(meth)acrylamide, N,N'-hexamethylenebis(meth)acrylamide, N-hydroxymethyl(meth)acrylamide, N-(2-hydroxyethyl)(meth)acrylamide, N,N-bis(2-hydroxyethyl)(meth)acrylamide, crotonic acid amide and cinnamic acid amide; (meth)acrylic acid esters such as methyl (meth)acrylate, ethyl (meth)acrylate, propyl (meth)acrylate, butyl (meth)acrylate, hexyl (meth)acrylate, lauryl (meth)acrylate, polyethylene glycol (meth)acrylate and polypropylene glycol (meth)acrylate; aromatic vinyl compounds such as styrene, α-methylstyrene, o-methoxystyrene, p-hydroxystyrene and p-isopropenylphenol; epoxy (meth)acrylates obtained by reacting diglycidyl ether of bisphenol A, diglycidyl ether of glycol or the like with (meth)acrylic acid, a hydroxyalkyl (meth)acrylate or the like; urethane (meth)acrylates obtained by reacting a hydroxyalkyl (meth)acrylate with polyisocyanate; epoxy group-containing unsaturated compounds such as glycidyl (meth)acrylate and (meth)allyl glycidyl ether; unsaturated acid compounds such as (meth)acrylic acid, itaconic acid, succinic acid-β-(meth)acryloxyethyl, maleic acid-β-(meth)acryloxyethyl and phthalic acid-β-(meth)acryloxyethyl; amino group-containing unsaturated compounds such as dimethylamino (meth)acrylate and diethylamino (meth)acrylate; and hydroxyl group-containing unsaturated compounds such as hydroxyethyl (meth)acrylate, hydroxypropyl (meth)acrylate and hydroxybutyl (meth)acrylate.

Among these additional monomers, butadiene, isoprene, (meth)acrylonitrile, (meth)acrylic acid alkyl esters, styrene, p-hydroxystyrene, p-isopropenylphenol, glycidyl (meth)acrylate, (meth)acrylic acid, hydroxyalkyl (meth)acrylates and the like are preferable.

Preferably, at least one diene compound, specifically butadiene, is used as the additional monomer for production of the above-mentioned crosslinked fine particles (D). The amount of such a diene compound is preferably from 20 to 80% by weight, more preferably from 30 to 70% by weight, especially preferably from 40 to 70% by weight, based on 100% by weight of all monomers to be used for copolymerization.

When the above-mentioned diene compound such as butadiene as additional monomer is copolymerized in an amount of 20 to 80% by weight based on 100% by weight of all monomers, rubber-like soft fine particles are formed as the crosslinked fine particles (D), so that the resulting cured film can be prevented from being cracked (broken), and a cured film excellent in durability can be obtained.

The crosslinked fine particles (D) may be used alone or in a mixture of two or more.

The average particle size of the crosslinked fine particles (D) is generally from 30 to 500 nm, preferably from 40 to 200 nm, more preferably from 50 to 120 nm.

A method of controlling the particle size of the crosslinked fine particles (D) is not particularly limited. For example, when the crosslinked fine particles are synthesized by emulsion polymerization, the number of micelles during the emulsion polymerization may be controlled by the amount of the emulsifying agent used so that the particle size can be controlled.

The average particle size of the crosslinked fine particles (D) is a value obtained by a process that includes diluting a dispersion of the crosslinked fine particles by a conventional method and measuring the dilution with a light scattering particle size analyzer or the like.

The amount of the crosslinked fine particles (D) formulated is preferably from 0.5 to 50 parts by weight, more preferably from 1 to 30 parts by weight, based on 100 parts by weight of the aforesaid phenolic resin (B). When the amount of the crosslinked fine particles (D) is from 0.5 to 50 parts by weight, they have excellent compatibility with other ingredients or excellent dispersibility, so that the resulting cured film can have improved thermal shock resistance and heat resistance.

### Adhesion auxiliary agent (E)

The chemically amplified negative photoresist composition of the invention may also contain an adhesion auxiliary agent for improving adhesion to the base material.

Examples of the above adhesion auxiliary agent include functional silane coupling agents having a reactive substituent such as a carboxyl group, a methacryloyl group, an isocyanate group, or an epoxy group.

The amount of the adhesion auxiliary agent formulated is preferably from 0.2 to 10 parts by weight, more preferably from 0.5 to 8 parts by weight, based on 100 parts by weight of the aforesaid phenolic resin (B). When the amount of the adhesion auxiliary agent formulated is from 0.2 to 10 parts by weight, excellent storage stability and good adhesion can be achieved, which is preferred.

### Solvent

The chemically amplified negative photoresist composition of the invention may also contain a solvent for improving the handleability of the resin composition or controlling viscosity or storage stability.

Specific examples of the above solvent include, but are not particularly limited to, those listed above.

The chemically amplified negative photoresist composition of the present invention may also contain a sensitizer if necessary. A conventionally known sensitizer may be used as such a sensitizer, specific examples of which include the aforementioned sensitizers.

The amount of the sensitizer used is from 5 to 500 parts by weight, preferably from 10 to 300 parts by weight, based on 100 parts by weight of the acid generator.

### Other Additives

If necessary, the chemically amplified negative photoresist composition of the invention may also contain an additional additive in such an extent that the characteristics of the invention are not impaired. Examples of such an additional additive include an inorganic filler, a sensitizer, a quencher, a leveling agent, and a surfactant.

A method for preparing the chemically amplified negative photoresist composition of the invention is not particularly limited, and it may be prepared by any known method. The composition may also be prepared by placing each ingredient in a sample vial, completely sealing the vial with a plug, and stirring the ingredients on a wave rotor.

The cured product of the present invention comprises a product obtained by curing the aforesaid chemically amplified negative photoresist composition.

The above-described chemically amplified negative photoresist composition of the present invention has high remaining thickness ratio and excellent resolution, and the cured product has excellent electric insulation, thermal shock resistance, heat resistance/colorability and the like. Therefore, the cured product is suitable for use as a surface protecting film, a planarizing film, an interlayer dielectric film material, or the like in electronic components such as semiconductor devices, transparent electrodes for displays, semiconductor packages and displays.

To form the cured product of the invention, the chemically amplified negative photoresist composition of the invention as described above is first applied to a support (such as a resin-bearing copper foil, a copper-clad laminate, a silicon wafer having a sputtered metal film, or an alumina substrate), and the solvent and the like are evaporated by drying, so that a coating film is formed. Subsequently, the coating film is exposed to light through a desired mask pattern and heat-treated (hereinafter the heat treatment is referred to as "PEB") so that the reaction between the phenolic resin (B) and the crosslinking agent (C) is accelerated. The unexposed part is then dissolved and removed by development with an alkaline developing solution, so that a desired pattern is obtained. Heat treatment for producing insulating film characteristics is further performed, so that a cured film is obtained.

An application method such as dipping, spraying, bar coating, roller coating, or spin coating may be used to apply the resin composition to the support. The thickness of the coating film can be appropriately controlled by controlling application means or the solids content or viscosity of a solution of the composition.

Examples of radiations for use in the exposure include ultraviolet rays from low-pressure mercury lamps, high-pressure mercury lamps, metal halide lamps, g-line steppers, h-line steppers, i-line steppers, gh-line steppers, or ghi-line steppers, electron beams, and laser beams. The exposure dose is appropriately selected depending on the light source used, the thickness of the resin film, or the like. For example, in the case of ultraviolet irradiation from a high-pressure mercury lamp, the exposure dose may be from about 100 to 50,000 J/m² for a resin film thickness of 1 to 50 µm.

After the exposure, the above PEB treatment is performed to accelerate the curing reaction between the phenolic resin (B) and the crosslinking agent (C), which is induced by the generated acid. The PEB conditions are generally 70 to 150°C, preferably 80 to 120°C, and about 1 to 60 minutes, although they depend on the amount of the resin composition formulated, the thickness of the film used, or the like. Subsequently, the unexposed part is dissolved and removed by development with an alkaline developing solution, so that a desired pattern is formed. In this case, the development method may be a shower development method, a spray development method, an immersion development method, a puddle development method, or the like. The development conditions are generally 20 to 40°C and about 1 to 10 minutes.

After the development, the film may be sufficiently cured by heat treatment so that insulting film characteristics can be sufficiently exerted. The curing conditions are not restricted. Depending on the intended use of the cured product, the composition may be cured by heating at a temperature of 50 to 250°C for about 30 minutes to 10 hours. Two-stage heating may also be performed so that the curing can sufficiently proceed or deformation of the resulting pattern shape can be prevented. For example, the curing at the first stage may be performed by heating at a temperature of 50 to 120°C for about 5 minutes to 2 hours, and the curing at the second state may be performed at a temperature of 80 to 250°C for about 10 minutes to 10 hours. Under such curing conditions, a general oven, an infrared furnace, or the like can be used as the heating equipment.

### EXAMPLES

Hereinafter, the invention is more specifically described with reference to Examples, which however are not intended to limit the invention.

### Example 1 Synthesis of lithium tetrakis(pentafluorophenyl) gallate

Charged were 500 mL of ultra-dehydrated diethyl ether and 30 g (121.46 mmol) of pentafluorobromobenzene in a 125 mL four-necked flask thoroughly dried under a nitrogen atmosphere, and the mixture was cooled to -78°C using a dry ice/acetone bath. Added dropwise was 47.4 mL of a 2.5 mol/L n-butyllithium hexane solution over 10 minutes, and the mixture was then stirred at -78°C for 30 minutes. Thereto was added dropwise 49.3 mL of a 0.6 mol/L gallium (III) chloride diethyl ether solution over 10 minutes, and the mixture was stirred at -78°C for 3 hours. The reaction liquid was stirred while being gradually returned to room temperature, and the reaction liquid was further stirred for 5 hours after being returned to room temperature. The precipitated solid was filtered, the reaction liquid was transferred to an evaporator, and the solvent was distilled off to obtain an off-white product. The product was washed with 30 mL of ultra-dehydrated hexane four times, and dried in vacuum overnight to obtain lithium tetrakis(pentafluorophenyl)gallate having a purity of 98% (yield: 87%).

### Reference example Synthesis of 4-isopropylphenyl(p-tolyl)iodonium tetrakis(pentafluorophenyl)gallate (purity: 90%)

Charged were 25 mL of dichloromethane, 1.69 g (5 mmol) of 4-isopropylphenyl(p-tolyl)iodonium chloride and 4.4 g (6 mmol) of lithium tetrakis(pentafluorophenyl)gallate in a 50 mL eggplant flask, and the mixture was stirred at room temperature for 2 hours. The precipitated solid was filtered, the reaction liquid was transferred to an evaporator, and the solvent was distilled off to obtain white 4-isopropylphenyl(p-tolyl)iodonium tetrakis(pentafluorophenyl)gallate having a purity of 90% (yield: 98%).

### Example 3 Synthesis of 4-isopropylphenyl(p-tolyl)iodonium tetrakis(pentafluorophenyl)gallate (purity: 96%)

Charged were 25 mL of dichloromethane, 1.69 g (5 mmol) of 4-isopropylphenyl(p-tolyl)iodonium chloride and 4.4 g (6 mmol) of lithium tetrakis(pentafluorophenyl)gallate in a 50 mL eggplant flask, and the mixture was stirred at room temperature for 2 hours. The precipitated solid was filtered, the dichloromethane layer was washed with water once in liquid separation operation, and then transferred to a rotary evaporator, and the solvent was distilled off to obtain white 4-isopropylphenyl(p-tolyl)iodonium tetrakis(pentafluorophenyl)gallate having a purity of 96% (yield: 96%).

### Example 4 Synthesis of 4-isopropylphenyl(p-tolyl)iodonium tetrakis(pentafluorophenyl)gallate (purity: 98%)

Charged were 25 mL of dichloromethane, 1.69 g (5 mmol) of 4-isopropylphenyl(p-tolyl)iodonium chloride and 4.4 g (6 mmol) of lithium tetrakis(pentafluorophenyl)gallate in a 50 mL eggplant flask, and the mixture was stirred at room temperature for 2 hours. The precipitated solid was filtered, the dichloromethane layer was washed with water twice in liquid separation operation, and then transferred to a rotary evaporator, and the solvent was distilled off to obtain white 4-isopropylphenyl(p-tolyl)iodonium tetrakis(pentafluorophenyl)gallate having a purity of 98% (yield: 93%).

### Example 5 Synthesis of 4-isopropylphenyl(p-tolyl)iodonium tetrakis(pentafluorophenyl)gallate (purity: 99.99%)

Charged were 25 mL of dichloromethane, 1.69 g (5 mmol) of 4-isopropylphenyl(p-tolyl)iodonium chloride and 4.4 g (6 mmol) of lithium tetrakis(pentafluorophenyl)gallate in a 50 mL eggplant flask, and the mixture was stirred at room temperature for 2 hours. The precipitated solid was filtered, the dichloromethane layer was washed with water twice in liquid separation operation, and transferred to a rotary evaporator, and the solvent was distilled off. The precipitated solid was added in 5 mL of dichloromethane to dissolve a white solid, and using a developing solvent of dichloromethane : hexane = 1 : 1, the solution was purified by column chromatography to obtain 4-isopropylphenyl(p-tolyl)iodonium tetrakis(pentafluorophenyl)gallate having a purity of 99.99% (yield: 85%).

### Example 6 Synthesis of 4-isopropylphenyl(p-tolyl)iodonium tetrakis(pentafluorophenyl)gallate (purity: 100%)

Charged were 25 mL of dichloromethane, 1.69 g (5 mmol) of 4-isopropylphenyl(p-tolyl)iodonium chloride and 4.4 g (6 mmol) of lithium tetrakis(pentafluorophenyl)gallate in a 50 mL eggplant flask, and the mixture was stirred at room temperature for 2 hours. The precipitated solid was filtered, the dichloromethane layer was washed with water twice in liquid separation operation, and transferred to a rotary evaporator, and the solvent was distilled off. The precipitated solid was recrystallized from dichloromethane-hexane to obtain white 4-isopropylphenyl(p-tolyl)iodonium tetrakis(pentafluorophenyl)gallate having a purity of 100% (yield: 50%).

### Example 7 Synthesis of lithium tetrakis(3,5-bis(trifluoromethyl)phenyl)gallate

Except that 30 g of pentafluorobromobenzene was replaced by 22.45 g of 1-bromo-3,5-bis(trifluoromethyl)benzene, the same procedure as in Example 1 was carried out to perform synthesis.

### Example 8 Synthesis of 4-isopropylphenyl(p-tolyl)iodonium tetrakis(3,5-bis(trifluoromethyl)phenyl)gallate

Except that 4.4 g of lithium tetrakis(pentafluorophenyl)gallate was replaced by 3.13 g of lithium tetrakis(3,5-bis(trifluoromethyl)phenyl)gallate, the same procedure as in Example 2 was carried out to perform synthesis.

### Example 9 Synthesis of 4-hydroxyphenyl-methyl-1-naphthylmethylsulfonium tetrakis(pentafluorophenyl)gallate

Dispersed was 1.59 g (5 mmol) of 4-hydroxyphenyl-methyl-1-naphthylmethylsulfonium chloride in 20 ml of dichloromethane, 41 g of an aqueous solution containing equimolar lithium tetrakis(pentafluorophenyl)gallate was mixed at room temperature, and the mixture was stirred as such for 3 hours. The dichloromethane layer was washed with water twice in liquid separation operation, and then transferred to a rotary evaporator, and the solvent was distilled off to obtain 4-hydroxyphenyl-methyl-1-naphthylmethylsulfonium tetrakis(pentafluorophenyl)gallate having a purity of 99% (yield: 91%).

### Example 10 Synthesis of 4-hydroxyphenyl-methyl-benzylsulfonium tetrakis(pentafluorophenyl)gallate

Dispersed was 1.59 g (5 mmol) of 4-hydroxyphenyl-methyl-benzylsulfonium chloride in 20 ml of dichloromethane, 15 g of an aqueous solution containing equimolar lithium tetrakis(pentafluorophenyl)gallate was mixed at room temperature, and the mixture was stirred as such for 3 hours. The dichloromethane layer was washed with water twice in liquid separation operation, and then transferred to a rotary evaporator, and the solvent was distilled off to obtain 4-hydroxyphenyl-methyl-benzylsulfonium tetrakis(pentafluorophenyl)gallate having a purity of 99% (yield: 90%).

### Example 11 Synthesis of 4-hydroxyphenyl-methyl-4-nitrobenzylsulfonium tetrakis(pentafluorophenyl)gallate

Dissolved were 1.08 g (5 mmol) of p-nitrobenzyl bromide and 0.7 g (5 mmol) of 4-(methyl)thiophenol in 15 ml of methanol, and the mixture was stirred at 50°C for 12 hours. Added were 30 ml of ion-exchanged water and 15 ml of ethyl acetate, the mixture was stirred for 30 minutes, and then separated, and the ethyl acetate layer was removed. Mixed were 37 g of an aqueous solution containing equimolar lithium tetrakis(pentafluorophenyl)gallate in an aqueous layer and 30 ml of ethyl acetate at room temperature, and the mixture was stirred as such for 3 hours. The ethyl acetate layer was washed with water twice in liquid separation operation, and the solvent was distilled off by a rotary evaporator to obtain 4-hydroxyphenyl-methyl-4-nitrobenzylsulfonium tetrakis(pentafluorophenyl)gallate having a purity of 97% (yield: 92%).

### Example 12 Synthesis of 4-hydroxyphenyldimethylsulfonium tetrakis(pentafluorophenyl)gallate

Dispersed was 0.96 g (5 mmol) of 4-hydroxyphenyldimethylsulfonium chloride in 20 ml of dichloromethane, 37 g of an aqueous solution containing equimolar lithium tetrakis(pentafluorophenyl)gallate was mixed at room temperature, and the mixture was stirred as such for 3 hours. The dichloromethane layer was washed with water twice in liquid separation operation, and then transferred to a rotary evaporator, and the solvent was distilled off to synthesize 4-hydroxyphenyldimethylsulfonium tetrakis(pentafluorophenyl)gallate having a purity of 99% (yield: 93%).

### Example 13 Synthesis of 4-acetoxyphenyldimethylsulfonium tetrakis(pentafluorophenyl)gallate

Dissolved was 4.47 g (5 mmol) of 4-hydroxyphenyldimethylsulfonium tetrakis(pentafluorophenyl)gallate synthesized in Example 8 in 50 ml of acetonitrile, 0.61 g (6 mmol) of triethylamine was added at 10°C or lower, and after 30 minutes, 0.47 g (6 mmol) of acetyl chloride was added dropwise. The mixture was stirred for 3 hours, a triethylamine hydrochloride generated as a by-product was then removed by filtration, the filtrate was transferred to a rotary evaporator, and the solvent was distilled off to obtain 4-acetoxyphenyldimethylsulfonium tetrakis(pentafluorophenyl)gallate having a purity of 98% (yield: 68.4%).

### Example 14 Synthesis of diphenyl[4-(phenylthio)phenyl]sulfonium tetrakis(pentafluorophenyl)gallate

Uniformly mixed were 1.6 g (8 mmol) of diphenyl sulfoxide, 1.5 g (8 mmol) of diphenyl sulfide, 2.5 g (24 mmol) of acetic anhydride, 1.5 g (10 mmol) of trifluoromethanesulfonic acid and 13 g of acetonitrile, and the mixture was reacted at 40°C for 6 hours. The reaction solution was cooled to room temperature, and poured into 60 g of distilled water. The mixture was extracted with 60 g of dichloromethane, and the dichloromethane layer was washed with water until the pH of the aqueous layer became neutral. The dichloromethane layer was transferred to a rotary evaporator, and the solvent was distilled off to obtain a brown liquid product. Thereto was added 20 g of ethyl acetate, and the mixture was dissolved in a water bath at 60°C. Subsequently, after 60 g of hexane was added and stirred, the operation of cooling the mixture to 5°C, allowing the mixture to stand for 30 minutes and then removing the supernatant was performed twice to wash the product. The product was transferred to a rotary evaporator, and the solvent was distilled off to obtain diphenyl[4-(phenylthio)phenyl]sulfonium triflate(triflate=trifluoromethanesulfonate anion) having a purity of 98% (yield: 85%).

### (Metathesis Process)

The triflate was dissolved in 50 g of dichloromethane, 66 g of an aqueous solution containing equimolar lithium tetrakis(pentafluorophenyl)gallate was mixed at room temperature, the mixture was stirred as such for 3 hours, the dichloromethane layer was washed with water twice in liquid separation operation, and then transferred to a rotary evaporator, and the solvent was distilled off to obtain diphenyl[4-(phenylthio)phenyl]sulfonium tetrakis(pentafluorophenyl)gallate having a purity of 99% (yield: 90%).
Comparative Example 1 Synthesis of 4-isopropylphenyl(p-tolyl)iodonium tetrakis(pentafluorophenyl)borate
   Dispersed was 3 g of 4-isopropylphenyl(p-tolyl)iodonium chloride in 10 g of water under stirring, 62 g of an aqueous solution containing equimolar sodium tetrakis(pentafluorophenyl)borate and 50 g of dichloromethane were poured under stirring, and the mixture was stirred and mixed at room temperature for 3 hours. The dichloromethane layer was washed with water twice in liquid separation operation, and then transferred to a rotary evaporator, and the solvent was distilled off to obtain 4-hydroxyphenylmethylbenzylsulfonium phenyltris(pentafluorophenyl)borate (yield: 92%).
Comparative Example 2 Synthesis of 4-isopropylphenyl(p-tolyl)iodonium hexafluoroantimonate
   Except that 62 g of an aqueous solution containing sodium tetrakis(pentafluorophenyl)borate was replaced by 24 g of an aqueous solution containing potassium hexafluoroantimonate, the same procedure as in Comparative Example 1 was carried out to perform synthesis.
Comparative Example 3 Synthesis of 4-isopropylphenyl(p-tolyl)iodonium hexafluorophosphate
   Except that 62 g of an aqueous solution containing sodium tetrakis(pentafluorophenyl)borate was replaced by 16 g of an aqueous solution containing potassium hexafluorophosphate, the same procedure as in Comparative Example 1 was carried out to perform synthesis.
Comparative Example 4 Synthesis of 4-hydroxyphenyl-methyl-benzylsulfonium tetrakis(pentafluorophenyl)borate
   Except that 41 g of an aqueous solution containing lithium tetrakis(pentafluorophenyl)gallate was replaced by 39 g of an aqueous solution containing tetrakis(pentafluorophenyl)borate, the same procedure as in Example 9 was carried out to perform synthesis.
Comparative Example 5 Synthesis of 4-hydroxyphenyl-methyl-benzylsulfonium hexafluoroantimonate
   Except that 41 g of an aqueous solution containing lithium tetrakis(pentafluorophenyl)gallate was replaced by 15 g of an aqueous solution containing potassium hexafluoroantimonate, the same procedure as in Example 9 was carried out to perform synthesis.
Comparative Example 6 Synthesis of 4-hydroxyphenyl-methyl-benzylsulfonium hexafluorophosphate
   Except that 41 g of an aqueous solution containing lithium tetrakis(pentafluorophenyl)gallate was replaced by 10 g of an aqueous solution containing potassium hexafluorophosphate, the same procedure as in Example 9 was carried out to perform synthesis.
Comparative Example 7 Synthesis of diphenyl[4-(phenylthio)phenyl]sulfonium tetrakis(pentafluorophenyl)borate
   Except that 66 g of an aqueous solution containing lithium tetrakis(pentafluorophenyl)gallate was replaced by 62 g of an aqueous solution containing tetrakis(pentafluorophenyl)borate, the same procedure as in Example 14 was carried out to perform synthesis.
Comparative Example 8 CPI-110A {diphenyl[4-(phenylthio)phenyl]sulfonium hexafluoroantimonate, manufactured by San-Apro Ltd.} was used as a comparative sulfonium salt.
Comparative Example 9 CPI-110P {diphenyl[4-(phenylthio)phenyl]sulfonium hexafluorophosphate, manufactured by San-Apro Ltd.} was used as a comparative sulfonium salt.

### [Evaluation]

### (Preparation of Heat- or Energy Ray-Curable Composition)

The acid generator in each of Reference example (example 2 in the Table 1) and examples 3 to 12 and 14 and Comparative Examples 1 to 9, a sensitizer (2,4-diethylthioxanthone), and an epoxide (3,4-epoxycyclohexylmethyl-3,4-epoxycyclohexane carboxylate, CELLOXIDE 2021 P manufactured by Daicel Chemical Industries, Ltd.) as a cationically polymerizable compound were uniformly mixed in an amount formulated as shown in Table 1 to prepare each of heat- or energy ray-curable compositions 2 to 11 of the present invention and comparative curable compositions 1 and 12 to 20.

**[Table 1]**

| No. | Examples of corresponding acid generator | Amount of acid generator formulated | Amount of sensitizer formulated | Epoxide |
|---|---|---|---|---|
| 1 | Example 2 | 2 | 2 | 100 |
| 2 | Example 3 | 2 | 2 | 100 |
| 3 | Example 4 | 2 | 2 | 100 |
| 4 | Example 5 | 2 | 2 | 100 |
| 5 | Example 6 | 2 | 2 | 100 |
| 6 | Example 8 | 2 | 2 | 100 |
| 7 | Example 9 | 2 | 0 | 100 |
| 8 | Example 10 | 2 | 0 | 100 |
| 9 | Example 11 | 2 | 0 | 100 |
| 10 | Example 12 | 2 | 0 | 100 |
| 11 | Example 14 | 2 | 0 | 100 |
| 12 | Comparative Example 1 | 2 | 2 | 100 |
| 13 | Comparative Example 2 | 2 | 2 | 100 |
| 14 | Comparative Example 3 | 2 | 2 | 100 |
| 15 | Comparative Example 4 | 2 | 0 | 100 |
| 16 | Comparative Example 5 | 2 | 0 | 100 |
| 17 | Comparative Example 6 | 2 | 0 | 100 |
| 18 | Comparative Example 7 | 2 | 0 | 100 |
| 19 | Comparative Example 8 | 2 | 0 | 100 |
| 20 | Comparative Example 9 | 2 | 0 | 100 |

### <Photosensitivity (Photo-Curability) Evaluation>

The energy ray-curable compositions 3, 6, 8 of the invention and comparative curable compositions 12-14, 18-20 obtained above was applied to a polyethylene terephthalate (PET) film using an applicator (40 µm). Using an ultraviolet irradiator, the PET film was irradiated with ultraviolet light whose wavelength was restricted with filters. The filters used were 365 Filter (manufactured by EYE GRAPHICS Co., Ltd., a filter for cutting off light with wavelengths of less than 365 nm), a filter for cutting off light with wavelengths of less than 340 nm). Forty minutes after the irradiation, the pencil hardness (JIS K 5600-5-4: 1999) of the coating film hardness was measured and evaluated according to the criteria below (the coating film had a thickness of about 40 µm after the curing).

### (Evaluation Criteria)

⊙: The pencil hardness is 2H or higher.
○: The pencil hardness is from H to B.
Δ: The pencil hardness is from 2B to 4B.
×: Due to liquidness or tackiness, it is not possible to measure the pencil hardness.

### (Ultraviolet Light Irradiation Conditions)

- Ultraviolet ray irradiator: belt conveyor-type UV irradiator (manufactured by EYE GRAPHICS Co., Ltd.)
- Lamp: 1.5 kW high-pressure mercury lamp
- Filters: 365 Filter (manufactured by EYE GRAPHICS Co., Ltd.)
- Irradiance (measured with a 365 nm head photometer): 100 mW/cm²
- Integral light dose (measured with a 365 nm head photometer): 300 mJ/cm²

### <Heat Resistance (Yellowing) Test-1>

The composition obtained as described above was applied onto a slide glass with a thickness of 40 µm by an applicator. Using an ultraviolet irradiator, the above-mentioned coated slide glass was irradiated with ultraviolet light.

### (Ultraviolet Light Irradiation Conditions)

- Ultraviolet ray irradiator: belt conveyor-type UV irradiator (manufactured by EYE GRAPHICS Co., Ltd.)
- Lamp: 1.5 kW high-pressure mercury lamp
- Irradiance (measured with a 365 nm head photometer): 100 mW/cm²
- Integral light dose (measured with a 365 nm head photometer): 1000 mJ/cm²

After the irradiation, the composition was cured at room temperature for 40 minutes, and then post-cured on a hot plate at 120°C for 30 minutes to prepare a heat resistance test sample.

The sample was heated for 15 minutes on a hot plate controlled to a temperature of 240°C, and the color of the coating film was visually evaluated. Evaluation criteria are as follows.

### (Evaluation Criteria)

⊙: Colorless (yellowing of coating film does not occur)
○: Pale yellow or yellow
×: Brown

**[Table 2]**

| No. | Synthesis Example | Onium gallate salt | | Photo-Curability | Heat resistance (yellowing) |
|---|---|---|---|---|---|
| | | Cation | Anion | | |
| 3 | Example 4 | IPTI (*1) | Ga(C₆F₅)₄ | ⊙ | ⊙ |
| 6 | Example 8 | IPTI | Ga((CF₃)₂C₆H₃)₄ | ⊙ | ⊙ |
| 8 | Example 10 | DPTS (*2) | Ga(C₆F₅)₄ | ⊙ | ⊙ |
| 12 | Comparative Example 1 | IPTI | B(C₆F₅)₄ | ⊙ | × |
| 13 | Comparative Example 2 | IPTI | SbF₆ | ⊙ | × |
| 14 | Comparative Example 3 | IPTI | PF₆ | × | ⊙ |
| 18 | Comparative Example 7 | DPTS | B(C₆F₅)₄ | ⊙ | × |
| 19 | Comparative Example 8 | DPTS | SbF₆ | ⊙ | × |
| 20 | Comparative Example 9 | DPTS | PF₆ | × | ⊙ |

| | | | | | |
|---|---|---|---|---|---|
| *1: IPTI; 4-isopropylphenyl(p-tolyl)iodonium *2: DPTS; diphenyl[4-(phenylthio)phenyl]sulfonium | | | | | |

### <Evaluation of Thermosensitivity (Thermal Curability)>

Each of the energy ray-curable compositions 7 to 10 of the present invention and the comparative curable compositions 14 to 16 obtained as described above was applied to a polyethylene terephthalate (PET) film using an applicator (40 µm). After the application, heating was performed at 100°C for 5 minutes using a hot plate, the pencil hardness (JIS K 5600-5-4: 1999) of the coating film hardness immediately after the heating was measured and evaluated according to the criteria below (the coating film had a thickness of about 40 µm after the curing).

### (Evaluation Criteria)

⊙: The pencil hardness is 2H or higher.
○: The pencil hardness is from H to B.
Δ: The pencil hardness is from 2B to 4B.
×: Due to liquidness or tackiness, it is not possible to measure the pencil hardness.

### <Heat Resistance (Yellowing) Test-2>

Each of the energy ray-curable compositions 3 to 6 of the present invention and the comparative curable compositions 11 to 13 obtained as described above was applied to a slide glass with a thickness of 40 µm by an applicator.

The sample was heated for 15 minutes on a hot plate controlled to a temperature of 240°C, and thus cured, and the color of the coating film was visually evaluated. Evaluation criteria are as follows.

### (Evaluation Criteria)

⊙: Colorless (yellowing of coating film does not occur)
○: Pale yellow or yellow
×: Brown

**[Table 3]**

| No. | Synthesis Example | Onium gallate salt | | Photo-Curability | Heat resistance (yellowing) |
|---|---|---|---|---|---|
| | | Cation | Anion | | |
| 7 | Example 9 | HPMNMS (*3) | Ga(C₆F₅)₄ | ⊙ | ⊙ |
| 8 | Example 10 | HPMBS (*4) | Ga(C₆F₅)₄ | ⊙ | ⊙ |
| 9 | Example 11 | HPMNBS (*5) | Ga(C₆F₅)₄ | ⊙ | ⊙ |
| 10 | Example 12 | APDMS (*6) | Ga(C₆F₅)₄ | ⊙ | ⊙ |
| 14 | Comparative Example 4 | HPMBS | B(C₆F₅)₄ | ⊙ | × |
| 15 | Comparative Example 5 | | SbF₆ | ⊙ | ⊙ |
| 16 | Comparative Example 6 | | PF₆ | × | ⊙ |

| | | | | | |
|---|---|---|---|---|---|
| *3: HPMNMS; 4-hydroxyphenyl-methyl-1-naphthylmethylsulfonium *4: HPMBS; 4-hydroxyphenyl-methyl-benzylsulfonium *5: HPMNBS; 4-hydroxyphenyl-methyl-4-nitrobenzylsulfonium *6: APDMS; 4-acetoxyphenyldimethylsulfonium | | | | | |

### <Photosensitivity (Photo-Curability) Evaluation-2>

The energy ray-curable compositions 1-5 obtained above were applied to a polyethylene terephthalate (PET) film using an applicator (40 µm). Using an ultraviolet irradiator, the PET film was irradiated with ultraviolet light whose wavelength was restricted with filters. The filters used were 365 Filter (manufactured by EYE GRAPHICS Co., Ltd., a filter for cutting off light with wavelengths of less than 365 nm), a filter for cutting off light with wavelengths of less than 340 nm). Forty minutes after the irradiation, the pencil hardness (JIS K 5600-5-4: 1999) of the coating film hardness was measured and evaluated according to the criteria below (the coating film had a thickness of about 40 µm after the curing).

### (Evaluation Criteria)

⊙: The pencil hardness is 2H or higher.
○: The pencil hardness is from H to B.
Δ: The pencil hardness is from 2B to 4B.
x: Due to liquidness or tackiness, it is not possible to measure the pencil hardness.

### (Ultraviolet Light Irradiation Conditions)

- Ultraviolet ray irradiator: belt conveyor-type UV irradiator (manufactured by EYE GRAPHICS Co., Ltd.)
- Lamp: 1.5 kW high-pressure mercury lamp
- Filters: 365 Filter (manufactured by EYE GRAPHICS Co., Ltd.)
- Irradiance (measured with a 365 nm head photometer): 100 mW/cm²
- Integral light dose (measured with a 365 nm head photometer): 300 mJ/cm²

### <Heat Resistance (Yellowing) Test-2>

The composition obtained as described above was applied onto a slide glass with a thickness of 40 µm by an applicator. Using an ultraviolet irradiator, the above-mentioned coated slide glass was irradiated with ultraviolet light.

### (Ultraviolet Light Irradiation Conditions)

- Ultraviolet ray irradiator: belt conveyor-type UV irradiator (manufactured by EYE GRAPHICS Co., Ltd.)
- Lamp: 1.5 kW high-pressure mercury lamp
- Irradiance (measured with a 365 nm head photometer): 100 mW/cm²
- Integral light dose (measured with a 365 nm head photometer): 1000 mJ/cm²

After the irradiation, the composition was cured at room temperature for 40 minutes, and then post-cured on a hot plate at 120°C for 30 minutes to prepare a heat resistance test sample.

The sample was heated for 15 minutes on a hot plate controlled to a temperature of 240°C, and the color of the coating film was visually evaluated. Evaluation criteria are as follows.

### (Evaluation Criteria)

⊙: Colorless (yellowing of coating film does not occur)
○: Pale yellow or yellow
×: Brown

**[Table 4]**

| No. | Synthesis Example | Content of impurities (Parts by weight) | Onium gallate salt | | Photo-Curability | Heat resistance (yellowing) |
|---|---|---|---|---|---|---|
| | | | Cation | Anion | | |
| 1 | Example 6 | 0 | IPTI (*1) | Ga(C₆F₅)₄ | ⊙ | ○ |
| 2 | Example 5 | 0.01 | | | ⊙ | ⊙ |
| 3 | Example 4 | 2 | | | ⊙ | ⊙ |
| 4 | Example 3 | 4 | | | ○ | ⊙ |
| 5 | Example 2 | 10 | | | Δ | ⊙ |

It is apparent from Tables 2, 3 and 4 that the curable composition including an onium gallate salt, which is obtained according to the present invention, is excellent in cationic polymerization initiation performance and heat resistance (yellowing).

### <Preparation and Evaluation of Negative Photoresist Composition>

As shown in Table 5, 1 part by weight of the ingredient (A) (acid generator), 100 parts by weight of the phenolic resin ingredient (B) (a copolymer (Mw=10,000) of p-hydroxystyrene/styrene=80/20 (in molar ratio)), 20 parts by weight of the crosslinking agent ingredient (C) (hexamethoxymethylmelamine (trade name: "NIKALAC MW-390", manufactured by Sanwa Chemical Co., Ltd.), 10 parts by weight of the crosslinked fine particles ingredient (D) (a copolymer comprising butadiene/acrylonitrile/hydroxybutyl methacrylate/methacrylic acid/divinylbenzene=64/20/8/6/2 (% by weight) (average particle size=65 nm, Tg=-38°C)), and 5 parts by weight of the adhesion auxiliary agent ingredient (E) (γ-glycidoxypropyltrimethoxysilane (trade name: "S510", manufactured by CHISSO CORPORATION)) were uniformly dissolved in 145 parts by weight of solvent-1 (ethyl lactate), so that the negative photoresist compositions 21 of the present invention and 22 of comparative composition were prepared.

**[Table 5]**

| No. | Synthesis Example | Acid generator (A) | | content of (A) | Resin component (B) | Crosslinking agent component (C) | Crosslinked particle (D) | Adhesion auxiliary agent ingredient (E) | Solvent -1 |
|---|---|---|---|---|---|---|---|---|---|
| | | Cation | Anion | | | | | | |
| 21 | Example 4 | IPTI (*1) | Ga(C₆F₅)₄ | 1 | 100 | 20 | 10 | 5 | 145 |
| 22 | Comparative Example 1 | | B(C₆F₅)₄ | 1 | 100 | 20 | 10 | 5 | 145 |

| | | | | | | | | | |
|---|---|---|---|---|---|---|---|---|---|
| *1: IPTI; 4-isopropylphenyl(p-tolyl)iodonium | | | | | | | | | |

### <Evaluation of UV Curability (Negative Resist)>

Each composition was applied to a silicon wafer substrate by spin coating and then dried by heating with a hot plate at 110°C for 3 minutes, so that a resin coating film with a thickness of about 20 µm was obtained. Subsequently, pattern exposure (i-line; 500 mJ/cm²) was performed using TME-150RSC (manufactured by TOPCON CORPORATION), and post-exposure baking (PEB) was performed with a hot plate at 110°C for 3 minutes. Subsequently, a developing treatment was performed for 2 minutes by an immersion method using an aqueous solution of 2.38% by weight tetramethylammonium hydroxide. The layer was washed with flowing water, and nitrogen was blown, so that a 50 µm line and space pattern was obtained.

In both the compositions 21 and 22, the remaining film ratio indicating the ratio of remaining film before and after development was 90% or more, and a favorable pattern shape was exhibited.

### <Heat Resistance (Yellowing) Test-3>

The silicon substrate with a pattern, which was obtained in the above-mentioned evaluation of UV curability, was heated for 15 minutes on a hot plate controlled to a temperature of 200°C, and the color of a pattern portion was visually evaluated. The results thereof are shown in Table 5. Evaluation criteria are as follows.

### (Evaluation Criteria)

⊙: Colorless (yellowing of coating film does not occur)
○: Pale yellow or yellow
×: Brown

**[Table 6]**

| No. | Synthesis Example | Acid generator (A) | | Heat resistance test (yellowing test) |
|---|---|---|---|---|
| | | Cation | Anion | |
| 21 | Example 4 | IPTI (*1) | Ga(C₆F₅)₄ | ⊙ |
| 22 | Comparative Example 1 | | B(C₆F₅)₄ | × |

It is apparent from Table 6 that the chemically amplified negative photoresist composition including an onium gallate salt, which is obtained according to the present invention, is excellent in UV curability and heat resistance (yellowing).

From the results in Tables 2, 3 and 6, it is apparent that the curable composition and the chemically amplified negative photoresist composition including an onium gallate salt, which are obtained according to the present invention, are useful for members that are required to have optical properties, such as displays, optical waveguides and optical lenses because favorable UV curability is exhibited, and a cured product has high transparency (hardly turns yellow) after a heat resistance test.

### INDUSTRIAL APPLICABILITY

The curable composition of the present invention is suitably used for paints, coating agents, various coating materials (hard coats, anti-fouling coating materials, anti-fogging coating materials, anti-corrosion coating materials, optical fibers and the like), back surface treatment agents for pressure sensitive adhesive tapes, release coating materials of release sheets for pressure sensitive adhesive labels (release papers, release plastic films, release metal foils and the like), printing plates, dental materials (dental formulations and dental composites), ink compositions, inkjet ink compositions, positive resists (for formation of connection terminals and wiring patterns in production of electronic components such as circuit boards, CSP and MEMS elements), resist films, liquid resists and negative resists (permanent film materials of surface protecting films, interlayer dielectric films, planarizing films for semiconductor elements, etc.), resists for MEMS, positive photosensitive materials, negative photosensitive materials, various adhesives (various temporary fixing agents for electronic components, adhesives for HDD, adhesives for pick-up lenses, functional films for FPD (polarizing plates, antireflection films and the like), etc.), holographic resins, FPD materials (color filters, black matrices, partition wall materials, photospacers, ribs, orientation films for liquid crystals, sealing agents for FPD and the like), optical members, molding materials (for building materials, optical components and lenses), casting materials, putty materials, glass fiber impregnating agents, fillers, sealing materials, sealants, photosemiconductor (LED) sealing materials, optical waveguide materials, nano-imprint materials, stereolithography materials, and micro-stereolithography materials.

## Claims

1. A heat- or energy ray-curable composition comprising a cationically polymerizable compound and an acid generator containing an onium gallate salt represented by general formula (1) below, wherein the gallate anion salt contains impurities in an amount of 4% or less: 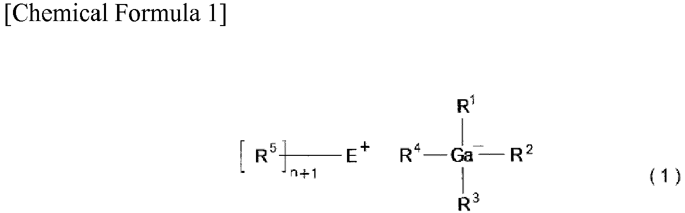
[in formula (1), R¹ to R⁴ each independently represent an alkyl group having 1 to 18 carbon atoms or Ar, with the proviso that at least one of R¹ to R⁴ is Ar;
Ar represents an aryl group having 6 to 14 carbon atoms (excluding the number of carbon atoms of substituents below), and some of hydrogen atoms in the aryl group may be substituted with an alkyl group having 1 to 18 carbon atoms, a halogen atom-substituted alkyl group having 1 to 8 carbon atoms, an alkenyl group having 2 to 18 carbon atoms, an alkynyl group having 2 to 18 carbon atoms, an aryl group having 6 to 14 carbon atoms, a nitro group, a hydroxyl group, a cyano group, an alkoxy group or aryloxy group represented by -OR⁶, an acyl group represented by R⁷CO-, an acyloxy group represented by R⁸COO-, an alkylthio group or arylthio group represented by -SR⁹, an amino group represented by -NR¹⁰R¹¹, or a halogen atom;
R⁶ to R⁹ each represent an alkyl group having 1 to 8 carbon atoms, or an aryl group having 6 to 14 carbon atoms;
R¹⁰ and R¹¹ each represent a hydrogen atom, an alkyl group having 1 to 8 carbon atoms, or an aryl group having 6 to 14 carbon atoms;
E represents an n-valent element of Group 15 to Group 17 (IUPAC nomenclature);
n represents an integer of 1 to 3; and
R⁵ is an organic group bonded to E, the number of R⁵s is n + 1, (n + 1) R⁵s may be the same or different, and two or more R⁵s may be linked together directly or through -O-, -S-, -SO-, -SO₂-, -NH-, -CO-, -COO-, -CONH-, an alkylene group or a phenylene group to form a ring structure including the element E].

2. The heat- or energy ray-curable composition according to claim 1, wherein the element E is S, I, N or P.

3. The heat- or energy ray-curable composition according to claim 1 or 2, wherein in general formula (1), R¹, R², R³ and R⁴ each represent a perfluoroalkyl group or a phenyl group substituted with a fluorine atom.

4. The heat- or energy ray-curable composition according to claim 1 or 2, wherein in general formula (1), R¹, R², R³ and R⁴ each represent a pentafluorophenyl group or a bis(trifluoromethyl)phenyl group.

5. The heat- or energy ray-curable composition according to claim 1 or 2, wherein in general formula (1), a gallate anion represented by [(R¹)(R²)(R³)(R⁴)Ga]⁻ is [Ga(C₆F₅)₄]⁻ or [Ga((CF₃)₂C₆H₃)₄]⁻.

6. The heat- or energy ray-curable composition according to any one of claims 1 to 5, wherein the cationically polymerizable compound is selected from epoxides and oxetanes.

7. A cured article obtained by curing the heat- or energy ray-curable composition according to any one of claims 1 to 6.

8. A chemically amplified negative photoresist composition comprising a heat- or energy ray-curable composition according to any one of claims 1 to 5, wherein the cationically polymerizable compound is an alkali-soluble resin (B) having a phenolic hydroxyl group, and comprising a crosslinking agent component (C).

9. The chemically amplified negative photoresist composition according to claim 8, further comprising crosslinked fine particles (D).

10. The chemically amplified negative photoresist composition according to claim 8 or 9, further comprising an adhesion auxiliary agent (E).

11. A cured article obtained by curing the chemically amplified negative photoresist composition according to any one of claims 8 to 10.

## Patentansprüche

1. Durch Wärme oder Energiestrahlung härtbare Zusammensetzung umfassend eine kationisch polymerisierbare Verbindung und einen Säurebildner enthaltend ein Onium-Gallatsalz gemäß der untenstehenden allgemeinen Formel (1), wobei das Gallatanionsalz Verunreinigungen in einer Menge von 4% oder weniger enthält: 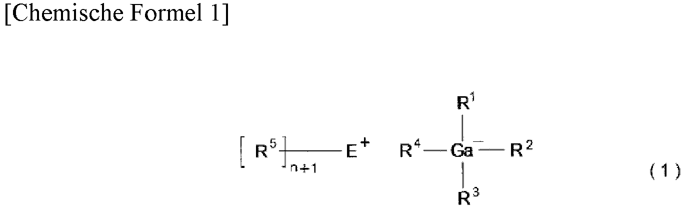
[in Formel (1) stellen R¹ bis R⁴ jeweils unabhängig voneinander eine Alkylgruppe mit 1 bis 18 Kohlenstoffatomen oder Ar dar, mit der Maßgabe, dass mindestens einer von R¹ bis R⁴ Ar ist;
Ar stellt eine Arylgruppe mit 6 bis 14 Kohlenstoffatomen (ausgenommen die Anzahl der Kohlenstoffatome der unten aufgeführten Substituenten) dar, wobei einige der Wasserstoffatome in der Arylgruppe mit einer Alkylgruppe mit 1 bis 18 Kohlenstoffatomen, einer mit einem Halogenatom substituierten Alkylgruppe mit 1 bis 8 Kohlenstoffatomen, einer Alkenylgruppe mit 2 bis 18 Kohlenstoffatomen, einer Alkynylgruppe mit 2 bis 18 Kohlenstoffatomen, einer Arylgruppe mit 6 bis 14 Kohlenstoffatomen, einer Nitrogruppe, einer Hydroxylgruppe, einer Cyanogruppe, einer Alkoxygruppe oder Aryloxygruppe gemäß -OR⁶, einer Acylgruppe gemäß R⁷CO-, einer Acyloxygruppe gemäß R⁸COO-, einer Alkylthiogruppe oder Arylthiogruppe gemäß -SR⁹, einer Aminogruppe gemäß -NR¹⁰R¹¹ oder einem Halogenatom substituiert sein können;
R⁶ bis R⁹ stellen jeweils eine Alkylgruppe mit 1 bis 8 Kohlenstoffatomen oder eine Arylgruppe mit 6 bis 14 Kohlenstoffatomen dar;
R¹⁰ und R¹¹ stellen jeweils ein Wasserstoffatom, eine Alkylgruppe mit 1 bis 8 Kohlenstoffatomen oder eine Arylgruppe mit 6 bis 14 Kohlenstoffatomen dar;
E stellt ein n-wertiges Element der Gruppe 15 bis Gruppe 17 (IUPAC-Nomenklatur) dar;
n stellt eine ganze Zahl von 1 bis 3 dar; und
R⁵ ist eine an E gebundene organische Gruppe, wobei die Anzahl von R⁵-Resten n + 1 beträgt, (n + 1) R⁵-Reste gleich oder verschieden sein können, und zwei oder mehr R⁵-Reste direkt oder durch -O-, -S-, -SO-, -SO₂-, -NH-, -CO-, -COO-, -CONH-, eine Alkylengruppe oder eine Phenylengruppe verbunden sein können, um eine das Element E enthaltende Ringstruktur zu bilden].

2. Durch Wärme oder Energiestrahlung härtbare Zusammensetzung gemäß Anspruch 1, wobei das Element E S, I, N oder P ist.

3. Durch Wärme oder Energiestrahlung härtbare Zusammensetzung gemäß Anspruch 1 oder 2, wobei in der allgemeinen Formel (1) R¹, R², R³ und R⁴ jeweils eine Perfluoralkylgruppe oder eine mit einem Fluoratom substituierte Phenylgruppe darstellen.

4. Durch Wärme oder Energiestrahlung härtbare Zusammensetzung gemäß Anspruch 1 oder 2, wobei in der allgemeinen Formel (1) R¹, R², R³ und R⁴ jeweils eine Pentafluorphenylgruppe oder eine Bis(trifluormethyl)phenylgruppe darstellen.

5. Durch Wärme oder Energiestrahlung härtbare Zusammensetzung gemäß Anspruch 1 oder 2, wobei in der allgemeinen Formel (1) ein Gallatanion, welches durch [(R¹)(R²)(R³)(R⁴)Ga]⁻ dargestellt wird, [Ga(C₆F₅)₄]⁻ oder [Ga((CF₃)₂C₆H₃)₄]⁻ ist.

6. Durch Wärme oder Energiestrahlung härtbare Zusammensetzung gemäß irgendeinem der Ansprüche 1 bis 5, wobei die kationisch polymerisierbare Verbindung aus Epoxiden und Oxetanen ausgewählt ist.

7. Gehärteter Gegenstand, welcher durch Härten der durch Wärme oder Energiestrahlung härtbaren Zusammensetzung gemäß irgendeinem der Ansprüche 1 bis 6 erhalten wird.

8. Chemisch amplifizierte Negativ-Photoresist-Zusammensetzung umfassend eine durch Wärme oder Energiestrahlung härtbare Zusammensetzung gemäß irgendeinem der Ansprüche 1 bis 5, wobei die kationisch polymerisierbare Verbindung ein alkalilösliches Harz (B) mit einer phenolischen Hydroxylgruppe ist, und umfassend eine Vernetzerkomponente (C).

9. Chemisch amplifizierte Negativ-Photoresist-Zusammensetzung gemäß Anspruch 8, welche ferner vernetzte Feinpartikel (D) umfasst.

10. Chemisch amplifizierte Negativ-Photoresist-Zusammensetzung gemäß Anspruch 8 oder 9, welche ferner ein Hafthilfsmittel (E) umfasst.

11. Gehärteter Gegenstand, der durch Härten der chemisch amplifizierten Negativ-Photoresist-Zusammensetzung gemäß irgendeinem der Ansprüche 8 bis 10 erhalten wird.

## Revendications

1. Composition durcissable à la chaleur ou aux rayons d'énergie comprenant un composé cationiquement polymérisable et un producteur d'acide contenant un sel de gallate d'onium représenté par la formule générale (1) ci-dessous, dans laquelle le sel d'anion de gallate contient des impuretés en une quantité de 4 % ou moins : 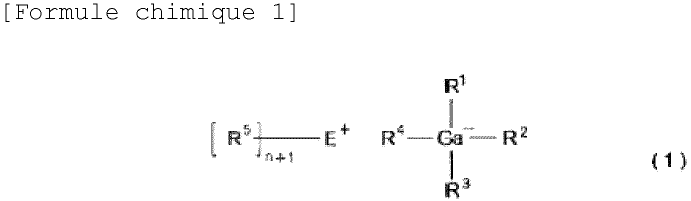
[dans la formule (1), R¹ à R⁴ représentent chacun indépendamment un groupe alkyle ayant 1 à 18 atomes de carbone ou Ar, à la condition qu'au moins un de R¹ à R⁴ soit Ar ;
Ar représente un groupe aryle ayant 6 à 14 atomes de carbone (à l'exclusion du nombre d'atomes de carbone des substituants ci-dessous) et certains des atomes d'hydrogène dans le groupe aryle peuvent être substitués avec un groupe alkyle ayant 1 à 18 atomes de carbone, un groupe alkyle substitué par un atome d'halogène ayant 1 à 8 atomes de carbone, un groupe alcényle ayant 2 à 18 atomes de carbone, un groupe alcynyle ayant 2 à 18 atomes de carbone, un groupe aryle ayant 6 à 14 atomes de carbone, un groupe nitro, un groupe hydroxyle, un groupe cyano, un groupe alcoxy ou un groupe aryloxy représenté par -OR⁶, un groupe acyle représenté par R⁷CO-, un groupe acyloxy représenté par R⁸COO-, un groupe alkylthio ou un groupe arylthio représenté par -SR⁹, un groupe amino représenté par -NR¹⁰R¹¹ ou un atome d'halogène ;
R⁶ à R⁹ représentent chacun un groupe alkyle ayant 1 à 8 atomes de carbone ou un groupe aryle ayant 6 à 14 atomes de carbone ;
R¹⁰ et R¹¹ représentent chacun un atome d'hydrogène, un groupe alkyle ayant 1 à 8 atomes de carbone ou un groupe aryle ayant 6 à 14 atomes de carbone ;
E représente un élément n-valent du groupe 15 au groupe 17 (nomenclature IUPAC) ;
n représente un nombre entier de 1 à 3 ; et
R⁵ est un groupe organique lié à E, le nombre de R⁵ est n + 1, (n + 1) R⁵ peuvent être identiques ou différents, et deux ou plus de deux R⁵ peuvent être liés ensemble directement ou par -O-, -S-, -SO-, -SO₂-, -NH-, - CO-, -COO-, -CONH-, un groupe alkylène ou un groupe phénylène pour former une structure cyclique comprenant l'élément E].

2. Composition durcissable à la chaleur ou aux rayons d'énergie selon la revendication 1, dans laquelle l'élément E est S, I, N ou P.

3. Composition durcissable à la chaleur ou aux rayons d'énergie selon la revendication 1 ou 2, dans laquelle dans la formule générale (1), R¹, R², R³ et R⁴ représentent chacun un groupe perfluoroalkyle ou un groupe phényle substitué avec un atome de fluor.

4. Composition durcissable à la chaleur ou aux rayons d'énergie selon la revendication 1 ou 2, dans laquelle dans la formule générale (1), R¹, R², R³ et R⁴ représentent chacun un groupe pentafluorophényle ou un groupe bis(trifluorométhyl)phényle.

5. Composition durcissable à la chaleur ou aux rayons d'énergie selon la revendication 1 ou 2, dans laquelle dans la formule générale (1), un anion de gallate représenté par [(R¹)(R²)(R³)(R⁴)Ga]⁻ est [Ga(C₆F₅)₄]⁻ ou [Ga((CF₃)₂C₆H₃)₄]⁻.

6. Composition durcissable à la chaleur ou aux rayons d'énergie selon l'une quelconque des revendications 1 à 5, dans laquelle le composé cationiquement polymérisable est choisi parmi des époxydes et des oxétanes.

7. Article durci obtenu en durcissant la composition durcissable à la chaleur ou aux rayons d'énergie selon l'une quelconque des revendications 1 à 6.

8. Composition photorésistante négative amplifiée chimiquement comprenant une composition durcissable à la chaleur ou aux rayons d'énergie selon l'une quelconque des revendications 1 à 5, dans laquelle le composé cationiquement polymérisable est une résine soluble aux alcali (B) ayant un groupe hydroxyle phénolique, et comprenant un composant d'agent de réticulation (C).

9. Composition photorésistante négative amplifiée chimiquement selon la revendication 8, comprenant en outre de fines particules réticulées (D).

10. Composition photorésistante négative amplifiée chimiquement selon la revendication 8 ou 9, comprenant en outre un agent auxiliaire d'adhésion (E).

11. Article durci obtenu en durcissant la composition photorésistante négative amplifiée chimiquement selon l'une quelconque des revendications 8 à 10.
